# EUROPEAN PATENT SPECIFICATION

(11) **EP 3 307 795 B1**
(45) Date of publication and mention of the grant of the patent: **13.03.2019**
(21) Application number: 16733320.2
(22) Date of filing: 13.06.2016
(51) Int. Cl.: C08F 2/22, C08L 1/02, C08L 3/02, C08L 3/04

(54) **STABLE RHEOLOGY MODIFIER COMPOSITIONS**
STABILE RHEOLOGIEMODIFIKATORZUSAMMENSETZUNGEN
COMPOSITIONS DE MODIFICATEUR DE RHÉOLOGIE STABLE

(30) Priority: 12.06.2015 US 201562174576 P; 11.12.2015 EP 15199606
(43) Date of publication of application: 18.04.2018
(73) Proprietor: Akzo Nobel Chemicals International B.V., 6824 BM Arnhem (NL)
(72) Inventor: RODRIGUES, Klin Aloysius, Signal Mountain, Tennessee 37377 (US); BAILEY, Andrew James, Chattanooga, Tennessee 37406 (US); VANDERHOOF, Matthew Michael, Hixson, Tennesse 37343 (US); PANTHA, Sajal, Ooltewah, Tennessee 37363 (US); ADAMO, Anthony John, Flagtown, New Jersey 08821-0215 (US); HAMM, Jaime Dion, Calabash, NC 28467 (US); SOLAREK, Daniel B., Hillsborough, NJ 08844 (US)
(74) Representative: Akzo Nobel Chemicals IP Group
(86) International application number: PCT/EP2016/063470
(87) International publication number: WO 2016/198688

(56) References cited:
- EP-A1- 1 099 712
- WO-A1-2014/139904
- WO-A1-2015/073117
- JP-A- H04 185 606

## Description

The invention relates to rheology modifier compositions with improved long term shelf life. More particularly, this invention relates to compositions of rheology modifiers having improved long term shelf life wherein the compositions are dried, the rheology modifiers comprising one or more swellable emulsion polymers and at least one water soluble support polymer.

### Background of the Invention

It is known in the art to provide rheology modifiers that are copolymers of ethylenically unsaturated carboxylic acid monomer (e.g., methacrylic acid) and ethylenically unsaturated ester monomer (e.g., ethyl acrylate), wherein one of the ethylenically unsaturated monomers is hydrophobic. When provided in acidic form, such copolymers are alkali swellable emulsion (ASE) copolymers that when neutralized can expand in water to act as a thickening agent. Such polymer emulsions are typically stabilized by surfactants, generally have a milky appearance, and when neutralized change from milky to clear. Commercially available ASE copolymer emulsions include Alcogum® L 11 available from Akzo Nobel Surface Chemistry LLC and Aqua SF-1 available from Lubrizol.

Emulsion polymerization is a process that can be considered to occur in a three-phase reaction system consisting of a continuous aqueous phase containing a dissolved initiator, large droplets of monomer (ca 1.5 microns dia), and colloidal particles (usually 50-150 nm dia) of monomer-swollen polymer. The initiator radical is formed in the aqueous phase. Essentially all of the polymerization reaction occurs in the monomer-polymer particles, with the monomer in the droplets being gradually transferred to the colloidal particles by dissolving in and diffusing through the aqueous phase. The monomer droplets and the swollen monomer-polymer particles are stabilized in the aqueous medium by absorbed surface-active agents, which are incorporated into the reaction mixture before the start of the polymerization. Chain transfer agents or modifiers can be added to the reaction mixture to limit the molecular weight. Emulsion polymerization is described in the Kirk-Othmer Encyclopedia of Chemical Technology, Vol. 18, 1982, pp. 742-743, incorporated herein by reference.

In a polysaccharide copolymer emulsion system, a combination of monomers (anionic or cationic ethylenically unsaturated monomer and hydrophobic ethylenically unsaturated monomer) reacts with the polysaccharide to form hydrophobically modified polysaccharide which constitutes the initial colloidal particles. After these particles are formed, the subsequent monomer that is added is polymerized in these particles.

In emulsion polymers that are stabilized by surfactants the initiator is soluble in the aqueous continuous phase but not soluble in the monomer phase. Also, the monomer mixture is not soluble in the aqueous continuous phase. Therefore, the initiator typically initiates in the aqueous phase. When the polymer fragment reaches a certain chain length, the fragment precipitates out into the droplets/particles and propagates further in the particles where more of the monomer is reacted and the polymer chain is terminated. This leads to higher molecular weight polymers, which can act as rheology modifiers by providing higher viscosities after neutralization. Therefore, it is important to have a minimum amount of hydrophobic ethylenically unsaturated monomer to precipitate the growing chain to form the emulsion polymerization system.

Dispersion polymerization is another technique for forming polymers that is distinct from emulsion polymerization. As described by Kawaguchi et al., Advances in Polymer Science, Volume 175, 2005, pp 299-328, incorporated herein by reference, dispersion polymerization is a type of precipitation polymerization in which a monomer which is soluble in the reaction medium is polymerized in the presence of an initiator and a suitable polymeric stabilizer which are also soluble in the reaction medium. The reaction medium is a good solvent for both the monomer and the steric stabilizer polymers, but is a non-solvent for the polymer being formed. Dispersion polymerization therefore involves a homogenous solution of monomer(s) with initiator and dispersant, in which sterically stabilized polymer particles are formed by the precipitation of the resulting polymers. Dispersion polymerization is a method for producing micron-size monodisperse polymer particles in a single batch process. By comparison, polymer particles produced by emulsion polymerization are much smaller, generally in the 100-500 nm range. In addition, the molecular weights of dispersion polymers are lower, resulting in polymers that generate less viscosity after neutralization.

US 4384096 discloses liquid emulsion polymers useful as pH responsive thickeners for aqueous systems, wherein the polymers prepared by the copolymerization of ethylenically unsaturated carboxylic acid monomer, ethylenically unsaturated non-ionic monomer, and non-ionic vinyl surfactant esters, to form an emulsion polymer stable as an aqueous colloidal dispersion.

US 4421902 discloses alkyl, poly(oxyethylene) poly(carbonyloxyethylene) acrylate emulsion copolymers for thickening purposes.

It is further known in the art to modify such copolymers by reaction with a small amount (generally < 1 mole%) of associative monomer having a pendant hydrophobe yielding a modified copolymer that provides improved thickening efficiency in water-based compositions. Such hydrophobically modified alkali swellable emulsion copolymers ("HASE" copolymers) when neutralized change from milky to clear solutions. One such HASE copolymer is sold by Akzo Nobel Surface Chemistry LLC under the product name Alcoguard® 5800.

Also known in the art are acid swellable emulsion copolymers that are copolymers of cationic ethylenically unsaturated monomers with hydrophobic ethylenically unsaturated monomer such as ethyl acrylate. These acid swellable emulsion copolymers also can be hydrophobically modified in a manner analogous to the HASE copolymers to make rheology modifiers. These cationic or acid swellable emulsion copolymers are used at pH of about 3-5, while the anionic or alkali swellable emulsion copolymers are used at pH of about 6-10. Acid swellable emulsions copolymers are sold by Akzo Nobel Surface Chemistry LLC under the product name Alcogum® L-520 and by Lubrizol under the product name Aqua CC.

Acid swellable emulsion copolymers will be stabilized, and alkali swellable emulsions copolymers will be optionally stabilized, by one or more surfactants. The stabilizing surfactants can be ionic, such as sodium lauryl sulfate, non-ionic, such as alcohol ethoxylates, or combinations thereof. The ionic surfactants can be anionic, cationic or amphoteric. Typically anionic monomers or a mixture of anionic and non-ionic monomers are used in alkali swellable emulsions, and cationic monomers or a mixture of cationic and non-ionic monomers are used in acid swellable emulsions.

Both acid-swellable emulsions and alkali swellable emulsions are sold as liquid compositions. One problem with all these types of swellable emulsion copolymers is their short shelf life. Acid swellable copolymer emulsions have a shelf life of about 3-6 months, while alkali swellable copolymer emulsions have a shelf life of about 6-9 months. Acid swellable emulsions are susceptible to hydrolysis and exhibit instability at higher storage temperatures. Furthermore, swellable emulsions are not freeze thaw stable. In addition, it would be desirable to have rheology modifiers that are at least partly biodegradable.

US 4,892,932 discloses a method for spray drying polymer emulsions and solutions wherein water is atomized together with the emulsion or solution to avoid unwanted polymer deposits. The reference acknowledges that the method requires a high energy demand, and therefore in practice it will be used only in those cases in which the dispersion without water addition can only be spray dried with difficulty or not at all.

EP 0398151A2 discloses copolymers of methyl methacrylate and methacrylic acid that can be spray dried. However, methyl methacrylate tends to form lower molecular weight copolymers than ethyl acrylate. This lowers the rheology modification performance and is not desired.

WO2014/139904A1 discloses a rheology modifier in which the acid and ester monomers are copolymerized in the presence of a polysaccharide to create polysaccharide alkali swellable emulsions. When neutralized these polymer emulsions change from milky to clear. In one embodiment the polysaccharide is a water-soluble starch. Advantageously these starch-modified copolymer emulsions do not require the use of a surfactant because the starch allows the emulsion to self-stabilize. These rheology modifiers are able to suspend active ingredients in a formulation, and also are partly biodegradable. But they have the same problems with short shelf life as the non-starch based copolymer emulsions.

For swellable emulsion polymers the drying process is significantly affected by the polymers' glass transition temperature. This is because upon drying swellable emulsion particles are forced into close contact with one another at which point they will either undergo coalescence, or not, based on the difference between the polymer's glass transition temperature relative to the drying temperature. Swellable emulsion polymers which undergo coalescence during drying typically form an intractable solid, which has a low probability of being usable, without significant further processing. Typical industrial drying processes may involve the application of significant heat to the material. This subjects these swellable emulsion polymers to temperatures that are above their glass transition temperature (Tg) which causes the swellable emulsion polymer particles to agglomerate, giving rise to either sticky films or intractable aggregates. For example, when attempting to spray dry a low glass transition temperature swellable emulsion polymer, the materials clog the nozzles of the spray dryer and form sticky films on the sides of the dryer.
It is known in the art that swellable emulsion polymers such as those described above will have glass transition temperatures of generally less than 50°C. Glass transition temperatures can be determined experimentally using known methods such as differential scanning calorimetry (DSC) or dynamic mechanical thermal analysis (DMTA), or a theoretically approximation can be obtained by use of the Fox equation (see T.G. Fox, Bull. Am. Phys. Soc. 1, 123 (1956)). It is well understood by those of ordinary skill in the art that there is a difference between the two values, yet an increase in Fox Tg typically correlates to an increase in measured Tg. Further, swellable emulsion polymers are significantly more hydrophilic in character than other emulsion polymers. Thus, even in cases where the Tg as calculated by use of the Fox equation is significantly above 50 °C it is known that hydrophilic swellable emulsion polymers as described herein may be significantly plasticized by water. This phenomenon, known as hydroplasticization, has been extensively studied and a methodology for the estimation of the extent of plasticization is reported by Sundberg and Tsavalas (Langmuir 2010, 26(10), 6960-6966). Dried rheology modifiers having a high water content are also disclosed in WO 2015/073117.

It would be desirable to provide swellable copolymers that can be used as rheology modifiers but which can be provided as solids, and in particular as free-flowing powders.

### Summary of the Invention

In accordance with the invention, a dried rheology modifier composition comprises at least one swellable emulsion polymer and at least one water soluble support polymer, wherein the at least one swellable emulsion polymer comprises at least one ethylenically unsaturated water soluble monomer and at least one ethylenically unsaturated hydrophobic monomer, wherein said dried rheology modifier composition comprises less than 25 wt% water. The dried rheology modifier composition can be dried by spray-drying.
The at least one swellable emulsion polymer can be an alkali swellable emulsion polymer.
The at least one swellable emulsion polymer can be an acid swellable emulsion polymer.
The at least one swellable emulsion polymer can further comprise a hydrophobic associative monomer.
The at least one water soluble support polymer can be a polysaccharide or a derivative thereof.
The at least one water soluble support polymer can be a polyvinyl acetate or derivative thereof.
The dried rheology modifier composition can be further heat treated after spray drying.

The dried rheology modifier composition can be heat treated and the water soluble support polymer may comprise a polysaccharide or a derivative thereof.

The at least one swellable emulsion polymer can be surfactant stabilized.

The at least one swellable emulsion polymer and the at least one water soluble support polymer can be connected by at least one chemical bond.

The at least one swellable emulsion polymer and the at least one water soluble support polymer can be blended together.

The invention further relates to a method of making a dried rheology modifier composition, the method comprising the steps of (i) providing a mixture of monomers and a water soluble support polymer, (ii) reacting the monomers that make up the alkali or acid swellable emulsion polymer in the presence of the water soluble support polymer to produce an alkali or acid swellable emulsion polymer composition, said reaction being optionally conducted in the presence of a surfactant, and (iii) drying said polymer composition.

Alternatively, the invention relates to a method of making a dried rheology modifier composition, the method comprising the steps of (i) providing a swellable emulsion polymer that is optionally surfactant stabilized, (ii) blending the swellable emulsion polymer with a water soluble support polymer, and (iii) drying the blend.

Optionally, in either of the methods of making the dried rheology modifier composition, prior to the drying step a second composition is added, the second composition comprising a second swellable emulsion polymer and optionally a second water soluble support polymer, wherein each of the second swellable emulsion polymer and the optional second water soluble support polymer of the second composition can be the same as or different from the at least one swellable emulsion polymer and at least one water soluble support polymer, respectively, of the initial polymer composition or blend.

The present invention also relates to product formulations comprising the dried rheology modifier compositions disclosed herein, and to the use of the dried rheology modifiers of the present invention as components of such product formulations. Such product formulations can be dry products, or can be liquid products into which the dried rheology modifier compositions of the present invention have been blended.

The dried rheology modifier compositions as disclosed herein have extended shelf life and improved freeze-thaw stability relative to emulsion rheology modifiers of the prior art. In addition, dried rheology modifier compositions of the present invention that have undergone a further heat treatment advantageously may impart a "short flow" rheology suitable for the formulation of gels and other products.

### Description of the Figures

Fig. 1 is a graph of the viscosities achieved in hair gel formulations using samples of a spray dried rheology modifier of the present invention that were heat treated for different lengths of time.
Fig. 2 is a graph showing the effect of temperature and duration of the heat treatment of rheology modifiers on the viscosity of gel formulations.
Fig. 3 is a graph showing high humidity curl retention achieved with a heat treated rheology modifier of the present invention compared to a control rheology modifier.

### Detailed Description of the Invention

The present invention relates to dried rheology modifier compositions comprising at least one swellable emulsion polymer and at least one water soluble support polymer. In one embodiment the rheology modifier composition can be dried by spray drying. The dried rheology modifier composition can be heat treated such that the rheology modifier composition imparts short flow rheological properties to a composition to which it is added.

The present invention further relates to methods of making rheology modifier compositions. One method comprises the steps of producing an alkali swellable or acid swellable emulsion polymer by reacting the monomers that make up the swellable emulsion polymer in the presence of a water soluble support polymer, and drying the resultant emulsion mixture. An alternative method comprises the steps of providing a swellable emulsion polymer, blending the swellable emulsion polymer with a water soluble support polymer, and drying the resultant emulsion mixture.

The invention is based on the surprising discovery that either (i) acid or alkali swellable emulsion polymers that have been produced by the emulsion polymerization of monomers in the presence of a water soluble support polymer, or (ii) acid swellable or alkali swellable emulsion polymers that are emulsion polymerized and then blended with a water soluble support polymer, can be dried, preferably by spray drying, to provide dried rheology modifier compositions in the form of stable powders. This invention also relates to the further surprising discovery that heat treating the dried rheology modifier compositions results in unexpected rheological properties. It has been found that heat treating the dried rheology modifier compositions results in products that exhibit a mucilage type rheology comparable to cross-linked polyacrylic acid (Carbomer) polymers. In addition, it has been found that the heat-treated compositions in accordance with the invention exhibit a higher viscosity in aqueous systems compared to the non-heat-treated versions.

The term "emulsion" as used herein refers to generally sub-micron sized polymer particles having a wide distribution of particle sizes and stabilized either by surfactants or by a water soluble support polymer. The at least one swellable emulsion polymer comprises at least one ethylenically unsaturated water soluble monomer and at least one ethylenically unsaturated hydrophobic monomer, wherein the hydrophobic monomer is water-insoluble. The acid or alkali swellable emulsion polymer is produced using an emulsion polymerization process. The emulsion particles have an average size of less than 1000 nm, preferably less than 750 nm and more preferably less than 500 nm. Emulsions are distinguished from polymer dispersions, in which the polymer particles are generally at least micron sized, have a narrower size distribution, and are produced from water soluble monomers generally in single batch processes.

The term "alkali swellable" as used herein means that a dried rheology modifier composition of the present invention that contains an alkali swellable emulsion polymer, when present in a solution as 2% solids of the swellable emulsion polymer, and neutralized to a pH of 7-8 with sodium hydroxide, can generate a viscosity at 25°C of about 500 mPas or more, preferably about 2500 mPas or more and more preferably about 5000 mPas or more when measured using a Brookfield Model DV-I viscometer at 10 rpm and 25C using an RV spindle #2. For example, if the dried rheology modifier composition contains 50 weight percent of an alkali swellable emulsion polymer and 50 weight percent water soluble support polymer, then a solution of 4 grams of dried rheology modifier in 100 grams of total aqueous solution would have 2% solids of the alkali swellable emulsion polymer portion.

The term "acid swellable" as used herein means that a dried rheology modifier compositions of the present invention that contains an acid swellable emulsion polymer, when present in a solution as 2% solids of the acid swellable emulsion polymer portion and neutralized to a pH of about 4-5 with glycolic acid, can generate a viscosity at 25°C of about 500 mPas or more, preferably about 1000 mPas or more and more preferably about 2500 mPas or more at 10 rpm at 25°C when measured using a Brookfield Model DV-I viscometer at 10 rpm and 25C using an RV spindle #2.

The term "water soluble support polymer" as used herein refers to a water soluble polymer that during and upon drying provides a polymeric rigid support for a dried product, such as a spray-dried powder.

With respect to the water soluble support polymers used in the compositions of the present invention, the term "water soluble" as used herein means about 0.5% or greater solubility in water at 25°C, in one embodiment about 5% or greater solubility in water at 25°C, and in one embodiment about 10% or greater solubility in water at 25°C (calculated on water plus water soluble support polymer weight basis).

The term "dried rheology modifier composition" as used herein means a composition comprising at least one swellable emulsion polymer and at least one water soluble support polymer, the composition being in a dry form comprising less than 25 wt% water, in one embodiment less than 20 wt% water, in one embodiment less than 10 wt% water, in one embodiment less than 5 wt% water, in one embodiment less than 2 wt% water, in one embodiment less than 1 wt% water, in one embodiment less than 0.5 wt% water.

All molecular weights as stated herein are weight average molecular weight unless stated otherwise.

The water soluble support polymer, when either present during the polymerization of an acid or alkali swellable emulsion polymer or blended with an acid or alkali swellable emulsion polymer, allows the resulting composition to be dried to produce a dried rheology modifier composition with less than 25 weight percent water. Without being bound by theory, it is believed that higher glass transition temperatures of the water soluble support polymer make it easier to dry the rheology modifier composition. In one embodiment the water soluble support polymer has a glass transition temperature of at least 50°C, in one embodiment at least 75°C, and in one embodiment at least 90°C.

Water soluble support polymers suitable for use in the present invention include polysaccharides such as starch and cellulose, and derivatives thereof. Other suitable water soluble support polymers include derivatives of polyvinyl acetate such as polyvinylalcohol, copolymers of polyvinyl alcohol/polyvinyl acetate, other copolymers of polyvinyl alcohol.

The weight percent of the water soluble support polymer for use with the present invention is at least about 20 weight % of the dried rheology modifier composition, preferably at least about weight 25% of the dried rheology modifier composition, and most preferably at least about 30 weight% of the dried rheology modifier composition. The maximum weight percent of the water soluble support polymer is no more than about 90 weight % of the dried rheology modifier composition, in another embodiment preferably no more than about 85 weight % of the dried rheology modifier composition, and in yet another embodiment most preferably no more than about 80 weight % of the dried rheology modifier composition.

In one embodiment of the dried rheology modifier composition, there is at least one chemical bond between the acid or alkali swellable emulsion polymer and the water soluble support polymer. This can be accomplished by conducting the polymerization of the acid or alkali swellable emulsion polymer in the presence of the water soluble support polymer.

In another embodiment the dried rheology modifier composition comprises a blend of the acid swellable or alkali swellable emulsion polymer with the water soluble support polymer.

In yet another embodiment of the invention, the dried rheology modifier composition comprises an acid or alkali swellable emulsion polymer that is both reacted and blended with one or more water soluble support polymers.

In yet another embodiment of the invention, the dried rheology modifier composition comprises more than one swellable emulsion copolymer.

In yet another embodiment of the invention, the dried rheology modifier comprises more than one water soluble support polymer.

The dried rheology modifier compositions of the present invention can be heat treated to develop internal crosslinks. The heat treatment can occur during the drying step where the temperature and time can be controlled. Alternatively, after the drying step the product can be further heated for a longer period of time under controlled temperatures.

In some product formulations it is desirable to have a flow behavior characterized as "long flow" behavior, similar to the flow of honey or syrup that runs off a spoon in a long thick stream, and in which an amount of the formulation placed on a flat surface spreads slowly and does not maintain its structural integrity over time. It is recognized in the art that such long flow behavior is not the same thing as a waterlike consistency which is characterized as "runny flow" behavior. In other product formulations it is desirable to have a flow behavior characterized as "short flow" behavior, similar to the flow of mayonnaise or gels that fall from a utensil in short drips or clumps and in which an amount of the formulation placed on a flat surface does not spread, and maintains its structural integrity over time. Generally, rheology modifier compositions of the present invention that have been dried but not subjected to further heat treatment are useful in product formulations wherein long flow behavior is desirable. Rheology modifier compositions of the present invention that have been dried and subjected to a further heat treatment step generally are useful in product formulations wherein short flow behavior is desirable; in such further heat treated rheology modifier compositions, at least one water soluble support polymer is a polysaccharide.

The term "heat treatment" as used herein means that the dried rheology modifier which contains a polysaccharide as the water soluble support polymer is subjected to a temperature of 100°C or greater for a time of one minute or longer.

Heat treatment is typically performed on dried compositions having low moisture, particularly less than about 25%, more particularly less than about 15%, most particularly less than about 1%. The heat treatment temperature is at least about 100°C, in some embodiments at least 110°C, and in some embodiments at least about 120°C, for a period of time of at least one minute, or at least five minutes, or at least about 30 minutes, or at least one hour, or at least 2 hours. Typically, the temperature is about 130°C for a period of less than about 4 hours.

The moisture content, pH, temperature and time of heat treatment may be adjusted to achieve the viscosity, dispersibility, gel texture, solution clarity, and other properties desired. However, at any given moisture, increasing the temperature or time generally increases viscosity to a maximum and further heating may decrease the viscosity. Heat treatment may be accomplished by a variety of methods known in the art including without limitation oven, fluidized bed, infrared and microwave heat treatments.

Optionally, in either of the methods of making the dried rheology modifier composition, prior to the drying step a second composition is added, the second composition comprising a second swellable emulsion polymer and optionally a second water soluble support polymer, wherein each of the second swellable emulsion polymer and the optional second water soluble protective polymer of the second composition can be the same as or different from the at least one swellable emulsion polymer and at least one water soluble support polymer, respectively, of the initial polymer composition or blend

In yet another embodiment of the invention different polysaccharides are dry blended with the dried rheology modifier composition and the blended composition is then heat treated. This allows for the inclusion in the final rheology modifier product of higher molecular weight polysaccharides that are otherwise more difficult to handle in aqueous compositions.

The particle size of the solid product may be adjusted using methods known in the art such as milling.

When added to a liquid formulation, the rheology modifier compositions of the invention unexpectedly develop enhanced thickening properties and, in some cases where the rheology modifier composition has been heat treated, impart to the formulation a gel-type short flow, instead of a honey-like long flow more typical of prior art rheology modifiers.

### Water Soluble Support Polymers

In one embodiment of the invention the water soluble support polymers are selected from polysaccharides.

Polysaccharides useful as the water soluble support polymers in the present invention can be derived from plant, animal and microbial sources. Examples of such polysaccharides include starch, cellulose, gums (e.g., gum arabic, guar and xanthan), alginates, pectin, carrageenan, inulin and gellan, and derivatives of each of the foregoing. One skilled in the art will recognize that the polysaccharides may need to depolymerized or derivatized to be water soluble. For example a starch may need to be depolymerized to a molecular weight of 10 million or less to be water soluble. Similarly a cellulose may have to be derivatized, for example to a carboxymethyl cellulose, to be water soluble.

The weight average molecular weight of the polysaccharide based water soluble support polymers can be about 20,000,000 or less, or 10,000,000 or less, or about 1,000,000 or less, or about 100,000 or less if it is reacted to form the dried emulsion rheology modifier. The weight average molecular weight of the polysaccharide based water soluble support polymers can be about 1,000,000 or less, or about 100,000 or less, or about 10,000 or less if it is blended to form the dried emulsion rheology modifier.

### Starch and Starch Derivatives

Starches include those derived from maize and conventional hybrids of maize, such as waxy maize and high amylose (greater than 40% amylose) maize, as well as other starches such as potato, tapioca, wheat, rice, pea, sago, oat, barley, rye, and amaranth, including conventional hybrids or genetically engineered materials. The starches can be of the native variety or a hybrid variety produced by traditional breeding programs or by artificial gene manipulation. These hybrids include but are not limited to waxy versions (starches with little or no amylose) and high amylose cultivars. Waxy starches are typically defined as having about 5% or less amylose and sometime containing about 2% or less amylose. In an embodiment, the waxy starches have about 95% or greater amylopectin. High amylose starches are defined as having about 40% or greater amylose (with the exception of pea starch which has a high amylose content of about 27% or greater amylose). In a further embodiment, the high amylose starches have an amylose content of about 60% or greater amylose. In addition, starches which have altered chain length and branch points are included in this application.

In an embodiment of the invention, the preferred polysaccharides are starches and starch derivatives, including, but not limited, to thermal and/or mechanically treated starch, oxidatively, hydrolytically or enzymatically degraded starches, and chemically modified starches. These preferred polysaccharides include maltodextrins, dextrin, pyrodextrins, oxidized starches, cyclodextrins and substituted cyclodextrins and higher molecular weight starches or derivatives thereof. In another preferred embodiment the preferred starches are waxy maltodextrins, waxy dextrins, waxy pyrodextrins, waxy oxidized starches, and higher molecular weight waxy starches or derivatives thereof. The most preferred starches are waxy maltodextrins. Chemical modification includes hydrolysis by the action of acids, enzymes, oxidizers or heat, esterification or etherification. The chemically modified starches, after undergoing chemical modification may be cationic, anionic, non-ionic or amphoteric or hydrophobically modified.

In an embodiment of the invention, the polysaccharide is a maltodextrin. Maltodextrins are polymers having d-glucose units linked primarily by α-1,4 bonds and a dextrose equivalent ('DE') of about 20 or less. Dextrose equivalent is a measure of the extent of starch hydrolysis. It is determined by measuring the amount of reducing sugars in a sample relative to dextrose (glucose). The DE of dextrose is 100, representing 100% hydrolysis. The DE value gives the extent of hydrolysis (e.g., 10 DE is more hydrolyzed than 5 DE maltodextrin). Maltodextrins are available as a white powder or concentrated solution and are prepared by the partial hydrolysis of starch with acid and/or enzymes.

Polysaccharides useful in the present invention can further include corn syrups. Corn syrups are defined as degraded starch products having a DE of 27 to 95. Examples of specialty corn syrups include high fructose corn syrup and high maltose corn syrup. Although not strictly polymers, monosaccharides and oligosaccharides such as galactose, mannose, sucrose, maltose, fructose, ribose, trehalose, lactose, etc., can be used as water soluble supports in the compositions and methods of the present invention, and for purposes hereof are considered to fall within the scope of water soluble support polymers.

In an embodiment of the invention, the polysaccharide has a DE of about 65 or less, 45 or less, 20 or less, in another embodiment a DE of about 15 or less and in still another embodiment a DE of about 5 or less. In an embodiment, the polysaccharide has a DE with a range having a lower limit of at least about 1.

In one embodiment of the invention, the polysaccharides are pregelatinized starch and its derivatives. The pregelatinized starches suitable for use in the present invention are those starches that have been treated with heat, moisture, or chemicals to disrupt the natural granular structure and render the starch soluble in water at below the gelatinization temperature of the native starch. For purposes of the invention, pregelatinized starches are also referred to as cold water soluble starches (CWS) and the terms are used interchangeably. For a general review of how to prepare pregelatinized starches see (Starch; Chemistry and Technology, R. L. Whistler, second edition, Academic Press, Inc. New York, 1984 pages 670 - 673). Additionally these products can be prepared by co-jet cooking coupled to a spray drier (see Kasica et al. US Patent 5,571,552). The pregelatinization can be done by different methods including but not limited to drum-drying, spray cooking or extrusion. In addition to being pregelatinized the starches of this invention can further be modified to contain anionic, cationic, non-ionic and reactive groups. Derivatives of these types are described in "Modified Starches: Properties and Uses" O.B. Wurzburg, CRC Press Boca Raton, Florida, 1986 chapters 3-9. The modified starches can be prepared in the granular form and then made cold water soluble or can be cooked before being reacted in solution to produce the polymers useful in this invention.

### Cellulose and cellulose derivatives

In an embodiment, the polysaccharides are celluloses and/or their derivatives such as carboxymethyl cellulose (CMC), hydroxethyl cellulose (HEC), carboxymethyl hydroxethyl cellulose (CMHEC), hydroxypropyl cellulose, sulfoethyl cellulose and its derivatives, ethyl hydroxyethyl cellulose (EHEC), methyl ethyl hydroxyethyl cellulose (MEHEC), and hydrophobically modified ethyl hydroxyethyl celluloses HM-EHEC some of which are available from AkzoNobel. Polysaccharides also include cellulosic derivatives, including plant heteropolysaccharides commonly known as hemicelluloses which are by-products of the paper and pulp industry. Hemicelluloses include xylans, glucuronoxylans, arabinoxylans, glucomannans, and xyloglucans. Xylans are the most common heteropolysaccharide and are preferred. Polysaccharides such as degradation products of cellulose such as cellobiose are suitable for preparing the polymers of this invention. Polysaccharides also include inulin and its derivatives, such as carboxymethyl inulin. In an embodiment, the preferred cellulosic materials are carboxymethyl cellulose (CMC), hydroxethyl cellulose (HEC), carboxymethyl hydroxethyl cellulose (CMHEC), hydroxypropyl cellulose, ethyl hydroxyethyl cellulose (EHEC), methyl ethyl hydroxyethyl cellulose (MEHEC), and hydrophobically modified ethyl hydroxy ethyl celluloses (HM-EHEC).

### Gums

Suitable polysaccharides include guar, unwashed guar gum, washed guar gum, cationic guar, carboxymethyl guar (CM guar), hydroxyethyl guar (HE guar), hydroxypropyl guar (HP guar), carboxymethylhydroxypropyl guar (CMHP guar), hydrophobically modified guar (HM guar), hydrophobically modified carboxymethyl guar (HMCM guar), hydrophobically modified hydroxyethyl guar (HMHE guar), hydrophobically modified hydroxypropyl guar (HMHP guar), cationic hydrophobically modified hydroxypropyl guar (cationic HMHP guar), hydrophobically modified carboxymethylhydroxypropyl guar (HMCMHP guar), hydrophobically modified cationic guar (HM cationic guar), guar hydroxypropyl triammonium chloride, hydroxypropyl guar hydroxypropyl triammonium chloride.

### Polyvinyl acetate derivatives

In one embodiment of the invention the water soluble support polymers are selected from water soluble derivatives of polyvinyl acetate such as polyvinylalcohol, copolymers of polyvinyl alcohol/polyvinyl acetate and copolymers of vinyl acetate and graft copolymers of polyvinyl alcohol/polyvinyl acetate. The derivatives of polyvinyl acetate can be anionic such as copolymers with anionic ethylenically unsaturated monomers or non-ionic when used with the alkali swellable emulsion polymers. The derivatives of polyvinyl acetate can be cationic such as copolymers with cationic ethylenically unsaturated monomers or non-ionic when used with the acid swellable emulsion polymers.

In one embodiment of the invention, the preferred polyvinyl acetate derivatives are completely or partially saponified and/or modified polyvinyl alcohols with a degree of hydrolysis of preferably approximately 70 to 100 mole %, and more preferably approximately 80 to 98 mole %. These polyvinyl alcohols have a Hoppler viscosity in 4% aqueous solution of preferably 1 to 50 mPas, in particular of approximately 3 to 40 mPas (measured at 20°C. according to DIN 53015). The weight average molecular weight of the polyvinyl acetate based water soluble support polymers can be about 1,000,000 or less or 500,000 or less, or about 100,000 or less if it is reacted to form the dried emulsion rheology modifier. The weight average molecular weight of the polyvinyl acetate based water soluble support polymers can be about 100,000 or less, or about 50,000 or less, or about 10,000 or less if it is blended to form the dried emulsion rheology modifier.

The support polymer is water soluble. For purposes of this invention, the polysaccharide has a solubility of at least 0.5 weight%, preferably at least 5 weight% and most preferably at least 10 weight percent in water at 25°C (calculated on water plus support polymer weight basis). In one embodiment, the water soluble support polymer is a starch that can be cooked in situ to make it water soluble during the reaction.

The use of polysaccharide as the water-soluble support polymer allows a significant portion of the rheology modifier compositions of the invention to be made from renewable resources, for products in which it is desirable to maximize the amount of renewable content.

Another advantage is that when the water soluble support polymer is present during the emulsion polymerization the amount of surfactant present during polymerization can be reduced; this is particularly so when the water soluble support polymer is a polysaccharide. Surfactant present during the emulsion polymerization of the present invention interferes with the reaction of the polysaccharide support polymer with the ethylenically unsaturated monomers. Therefore it is desirable to minimize or even eliminate the use of surfactant during the emulsion polymerization. This is distinct from the prior use of polysaccharides as a protective colloid. In protective colloid systems the goal is to minimize the amount of the protective colloid to minimize its effect on the properties of the final product. Also, in protective colloid polymerization systems a surfactant helps stabilize the micelles during polymerization, and improves shelf stability of the final product. Therefore, a significant amount of surfactant must be used with the protective colloid. For example, it is typical to use 0.1 to 1% surfactant based on the weight of the polymer in a typical protective colloid stabilized emulsion polymerization. However, in the emulsion polymerization of the present invention this amount of surfactant could interfere with the reaction of the polysaccharide and the emulsion polymer. Therefore, in the present invention it is preferable to have less than 0.1% surfactant or less, based on the weight of the emulsion polymer.

### Swellable Emulsion Polymers

The swellable emulsion polymers used in the present invention comprise at least one ethylenically unsaturated water soluble monomer and at least one ethylenically unsaturated water insoluble hydrophobic monomer. Where the at least one ethylenically unsaturated water soluble monomer is anionic then the emulsion polymer is alkali swellable. Where the at least one ethylenically unsaturated water soluble monomer is cationic then the emulsion polymer is acid swellable. Both alkali swellable emulsion polymers and acid swellable emulsion polymers can be hydrophobically modified by further comprising a hydrophobic associative monomer.

### Alkali Swellable Emulsion Polymers

Alkali swellable emulsion polymers useful in the inventive rheology modifier compositions of the present invention comprise at least one anionic ethylenically unsaturated monomer that is water soluble and at least one hydrophobic ethylenically unsaturated monomer that is water insoluble and, optionally, an associative monomer.

As used herein, the term "anionic ethylenically unsaturated monomer" means an ethylenically unsaturated monomer which is capable of developing a negative charge when the alkali swellable rheology modifier is in an aqueous solution. These anionic ethylenically unsaturated monomers can include, but are not limited to, acrylic acid, methacrylic acid, ethacrylic acid, α-chloro-acrylic acid, α-cyano acrylic acid, *β*-methyl-acrylic acid (crotonic acid), α-phenyl acrylic acid, *β*-acryloxy propionic acid, sorbic acid, *α*-chloro sorbic acid, angelic acid, cinnamic acid, p-chloro cinnamic acid, *β*-styryl acrylic acid (1-carboxy-4-phenyl butadiene-1,3), itaconic acid, maleic acid, citraconic acid, mesaconic acid, glutaconic acid, aconitic acid, fumaric acid, tricarboxy ethylene, muconic acid, 2-acryloxypropionic acid, 2-acrylamido-2-methyl propane sulfonic acid, vinyl sulfonic acid, sodium methallyl sulfonate, sulfonated styrene, allyloxybenzene sulfonic acid, vinyl phosphonic acid and maleic acid. Combinations of anionic ethylenically unsaturated monomers can also be used. In an embodiment of the invention, the anionic ethylenically unsaturated monomer may preferably be methacrylic acid, maleic acid, acrylic acid, itaconic acid, 2-acrylamido-2-methyl propane sulfonic acid or mixtures thereof. In an embodiment, most preferably the anionic ethylenically unsaturated monomer is methacrylic acid or acrylic acid, or combinations thereof.

The anionic ethylenically unsaturated monomer is water soluble. For purposes of this invention, the anionic ethylenically unsaturated monomer is "water soluble" as used herein means about 3.5% or greater solubility in water at 25°C, in one embodiment about 5% or greater solubility in water at 25°C, and in one embodiment about 10% or greater solubility in water at 25°C (calculated on water plus monomer weight basis).

For purposes of the present invention, the term "hydrophobic ethylenically unsaturated monomer" means an ethylenically unsaturated monomer that is hydrophobic and results in the formation of an emulsion system when reacted with the anionic ethylenically unsaturated monomer. For purposes of this invention, a hydrophobic ethylenically unsaturated monomer is considered "water-insoluble" where it has a water solubility of less than 3% at 25°C and most preferably less than 2.5% at 25°C (calculated on water plus monomer weight basis). These hydrophobic monomers may contain linear or branched alk(en)yl, cycloalk(en)yl, aryl, or alk(en)aryl moieties. Suitable hydrophobic ethylenically unsaturated monomers include C₁-C₇ alkyl esters or amides of acrylic and methacrylic acid including ethyl (meth)acrylate, methyl (meth)acrylate, butyl (meth)acrylate, styrene, vinyltoluene, t-butyl styrene, isopropylstyrene, and p-chlorostyrene; vinyl acetate, vinyl butyrate, vinyl caprolate, acrylonitrile, methacrylonitrile, butadiene, isobutylene, isoprene, vinyl chloride, vinylidene chloride, tertiary butyl acrylamide, benzyl (meth)acrylate, phenyl (meth)acrylate, benzyl ethoxylate (meth)acrylate, phenyl ethoxylate (meth)acrylate, 2-ethylhexyl (meth)acrylate, 2-butyloctyl (meth)acrylate, 2-hexyldecyl (meth)acrylate, 2-octyldodecyl (meth)acrylate, 2-decyltetradecyl (meth)acrylate, 2-dodecylhexadecyl (meth)acrylate, isopropyl (meth)acrylate, isobutyl (meth)acrylate, tertiary butyl (meth)acrylate, t-octyl acrylamide, octyl acrylate, lauryl acrylate, stearyl acrylate, behenyl acrylate, 2-ethylhexyl methacrylate, octyl methacrylate, lauryl methacrylate, stearyl methacrylate, behenyl methacrylate, 2-ethylhexyl acrylamide, n-octyl acrylamide, lauryl acrylamide, stearyl acrylamide, behenyl acrylamide, propyl acrylate, butyl acrylate, pentyl acrylate, hexyl acrylate, 1-allyl naphthalene, 2-allyl naphthalene,1-vinyl naphthalene, 2-vinyl naphthalene. and combinations thereof. Preferred are ethyl (meth)acrylate, methyl (meth)acrylate, 2-ethylhexyl acrylate, butyl (meth)acrylate, tertiary butyl acrylamide and combinations thereof. In an embodiment, ethyl acrylate, methyl acrylate, methyl methacrylate, butyl acrylate and combinations thereof are preferred. Vinyl acetate has a tendency to hydrolyze on storage and is not preferred. Vinyl acetate may be used in combination with other hydrophobic ethylenically unsaturated monomers such as ethyl acrylate, methyl acrylate, methyl methacrylate and butyl acrylate. When vinyl acetate is used, it is preferably less than 50 weight percent of the total hydrophobic ethylenically unsaturated monomer and more preferably less than 30 weight percent of the total hydrophobic ethylenically unsaturated monomer and most preferably less than 10 weight percent of the total hydrophobic ethylenically unsaturated monomer.

For purposes of the present invention, an associative monomer is intended to mean an ethylenically unsaturated monomer containing a hydrophobe and a spacer moiety which allows the hydrophobe to be sufficiently far away from the backbone of the polymer to form hydrophobic associations in aqueous solutions. The spacer moieties are usually ethoxylate groups but any other group that extends the hydrophobe away from the backbone of the polymer may be used. The hydrophobes with a spacer moiety include, but are not limited to, alcohol ethoxylates, alkylphenoxy ethoxylates, propoxylated/butoxylated ethoxylates, ethoxylated silicones and the like. In an embodiment, the preferred hydrophobes with spacer moieties include alcohol ethoxylates and/or alkylphenoxy ethoxylates. In another embodiment, alcohol ethoxylates containing alcohols with carbon chain lengths of 6 to 40 and having 6 to 100 moles of ethoxylation are more preferred. In yet another embodiment, alcohol ethoxylates containing alcohols with carbon chain lengths of 12 to 22 and 15 to 30 moles of ethoxylation are particularly preferred. The hydrophobes may be linear or branched alk(en)yl, cycloalk(en)yl, aryl, alk(en)aryl or an alkoxylated derivative. In an embodiment, the most preferred hydrophobes are linear or branched alcohols and amines containing 12 to 32 carbons. The associative monomer may contain an ethylenically unsaturated monomer covalently linked to the hydrophobe. In an embodiment, the ethylenically unsaturated monomer part of the associate monomer preferably is a (meth)acrylate, itaconate and/or maleate which contains ester linking groups. However, the associative monomer may also contain amide, urea, urethane, ether, alkyl, aryl and other suitable linking groups. The hydrophobe may be an alkylamine or dialkylamine ethoxylate. In an embodiment, the (meth)acrylate group is most preferred. In another embodiment, preferred associative monomers are C₁₂₋₃₂(EO)₁₀₋₃₀ meth(acrylates) or C₁₂₋₃₂(EO)₁₀₋₃₀ itaconates or C₁₂₋₃₂(EO)₁₀₋₃₀ maleates.

In one embodiment, the associative monomer has the structure 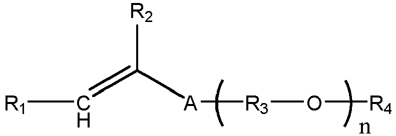
R₁ is -H, -CH₃, -COOH, or -CH₂COOH;
A is -CH₂C(O)O-, -C(O)O-, -O-, -CH₂O-, -CH₂C(O)N-, -C(O)N-, -CH₂-, -O-C(O)-,-NHC(O)O-, -NHC(O)NH-, -C₆H₄(R₅)-NH-C(O)-O-, -C₆H₄(R₅)-NH-C(O)-NH-,-C(O)O-CH₂-CH(CH₂OH)-O-, -C(O)O-CH₂-CH(CH₂OH)-NH-, -C(O)O-CH₂-CH-CH₂(OH)-O-, -C(O)O-CH₂-CH-CH₂(OH)-NH-, -CH₂-O-CH₂-CH(CH₂OH)-O-, -CH₂-O-CH₂-CH-CH₂(OH)-O-, -CH₂-O-CH₂-CH(CH₂OH)-NH-, or -CH₂-O-CH₂-CH-CH₂(OH)-NH-;
(R₃-O)ₙ is a polyoxyalkylene, which is a homopolymer, a random copolymer, or a block copolymer of C₂ to C₄ oxyalkylene units, wherein each R₃ is independently - C₂H₄-, -C₃H₆-, -C₄H₈-, or a mixture thereof, and n is an integer in the range of about 5 to about 250, preferably, n is 5-100, more preferably 10-50 and most preferably 15-30 ;
R₄ is C₆-C₃₆ linear or branched, saturated or unsaturated alk(en)yl or alk(en)aryl, most preferably C₈-C₃₂ linear or branched alk(en)yl, more preferably C₁₀-C₂₂ linear alk(en)yl or C₁₀-C₃₂ branched alk(en)yl; and
R₅ is CH₂ or (C)(CH₃)₂.

The anionic ethylenically unsaturated monomer can be present at about 15 weight percent or more of the total monomer used to produce the alkali swellable emulsion polymer, preferably about 20 weight percent or more of the total monomer used to produce the alkali swellable emulsion polymer, and most preferably about 30 weight percent or more of the total monomer used to produce alkali swellable emulsion polymer. The maximum weight of the anionic ethylenically unsaturated monomer can be about 80 weight percent or less of the total monomer used to produce the alkali swellable emulsion polymer, preferably about 70 weight percent or less of the total monomer used to produce the alkali swellable emulsion polymer, and most preferably about 60 weight percent or less of the total monomer used to produce alkali swellable emulsion polymer.

The minimum amount of hydrophobic ethylenically unsaturated monomer required is an amount effective to form an emulsion, which may depend on the hydrophobicity of the monomer. Generally, the higher the hydrophobicity the less monomer will be required to form an emulsion. The minimum amount of the hydrophobic ethylenically unsaturated monomer effective to form an emulsion can be about 10 weight percent or more of the total monomer used to produce the alkali swellable emulsion polymer, preferably about 25 weight percent or more of the total monomer used to produce the alkali swellable emulsion polymer, and most preferably about 40 weight percent or more of the total monomer used to produce the alkali swellable emulsion polymer. The maximum amount of the hydrophobic ethylenically unsaturated monomer is about 95 weight percent or less of the total monomer used to produce the alkali swellable emulsion polymer, preferably about 90 weight percent or less of the total monomer used to produce the alkali swellable emulsion polymer, and most preferably about 80 weight percent or less of the total monomer produce the alkali swellable emulsion polymer.

In some cases the use of styrene or substituted styrene in the production of swellable emulsion polymers may lead to high residual monomer levels which cause undesirable odors. Accordingly, in an embodiment of the invention, if styrene or substituted styrene is included as one part of the hydrophobic ethylenically unsaturated monomer, then the amount of this monomer can be about 10 weight percent or less of the total monomer, or about 5 weight percent or less of the total monomer or about 1 weight percent or less of the total monomer.

Use of the associative monomer is optional. When used, the minimum amount of the associative monomer can be about 0.1 weight percent or more of the total monomer used to produce the alkali swellable emulsion polymer, preferably about 1 weight percent or more of the total monomer used to produce the alkali swellable emulsion polymer, and most preferably about 2 weight percent or more of the total monomer used to produce the alkali swellable emulsion polymer. The maximum amount of the associative monomer can be about 30 weight percent or less of the total monomer used to produce the alkali swellable emulsion polymer, in another embodiment preferably about 25 weight percent or less of the total monomer used to produce the alkali swellable emulsion polymer, and in yet another embodiment most preferably about 20 weight percent or less of the total used to produce the alkali swellable emulsion polymer.

Further by way of example, dried rheology modifier compositions of the present invention that contain an alkali swellable emulsion polymer, and neutralized to a pH of 7-8 with sodium hydroxide, may have a water solubility of at least 0.1 weight percent at 25°C. In another embodiment, the dried rheology modifier compositions that contain an alkali swellable emulsion polymer, and neutralized to a pH of 7-8 with sodium hydroxide, may have a water solubility of at least 0.5 weight percent at 25°C. In another embodiment, the dried rheology modifier compositions that contain an alkali swellable emulsion polymer, and neutralized to a pH of 7-8 with sodium hydroxide, may have a water solubility of at least 1 weight percent at 25°C. In another embodiment, the dried rheology modifier compositions that contain an alkali swellable emulsion polymer, and neutralized to a pH of 7-8 with sodium hydroxide, may have a water solubility of at least 2 weight percent at 25°C.

It is to be understood that the foregoing is for example purposes only, that many other organic and inorganic bases are known to be useful in neutralizing alkali swellable emulsion polymers, and that the utility of rheology modifier compositions of the present invention comprising the alkali swellable emulsion polymers is not limited to formulations having pH of 7-8 or that are neutralized with sodium hydroxide.

### Acid Swellable Emulsion Polymers

Acid swellable emulsion polymers useful in the dried rheology modifier compositions of the present invention comprise at least one cationic ethylenically unsaturated monomer that is water soluble and at least one hydrophobic ethylenically unsaturated monomer that is water insoluble and, optionally, an associative monomer.

As used herein, the term "cationic ethylenically unsaturated monomer" means an ethylenically unsaturated monomer which is capable of developing a positive charge when the acid swellable emulsion polymer is in an aqueous solution. In an embodiment of the present invention, the cationic ethylenically unsaturated monomer has at least one amine functionality. As used herein, the term "amine salt" means that the nitrogen atom of the amine functionality is covalently bonded to from one to three organic groups and is associated with an anion.

These cationic ethylenically unsaturated monomers include, but are not limited to, N,N dialkylaminoalkyl(meth)acrylate, N-alkylaminoalkyl(meth)acrylate, N,N dialkylaminoalkyl(meth)acrylamide and N - alkylaminoalkyl(meth)acrylamide, where the alkyl groups are independently C₁-₁₈ linear, branched or cyclic moieties. Aromatic amine containing monomers such as vinyl pyridine may also be used. Furthermore, acyclic monomers such as vinyl formamide, vinyl acetamide and the like which generate amine moieties on hydrolysis may also be used. Preferably the cationic ethylenically unsaturated monomer is selected from one or more of N,N-dimethylaminoethyl methacrylate, tert-butylaminoethylmethacrylate, N,N-dimethylaminopropyl methacrylamide, 3-(dimethylamino)propyl methacrylate, 2 - (dimethylamino)propane-2-yl methacrylate, 3-(dimethylamino)-2,2-dimethylpropyl methacrylate, 2-(dimethylamino)-2-methylpropyl methacrylate and 4-(dimethylamino)butyl methacrylate and mixtures thereof. The most preferred cationic ethylenically unsaturated monomers are N,N-dimethylaminoethyl methacrylate, tert-butylaminoethylmethacrylate and N,N-dimethylaminopropyl methacrylamide.

The cationic ethylenically unsaturated monomer is preferably water soluble. For purposes of this invention, the cationic ethylenically unsaturated monomer is "water soluble" as used herein means about 3.5% or greater solubility in water at 25°C, in one embodiment about 5% or greater solubility in water at 25°C, and in one embodiment about 10% or greater solubility in water at 25°C (calculated on water plus monomer weight basis).

The cationic ethylenically unsaturated monomer can be present at about 15 weight percent or more of the total monomer used to produce the acid swellable emulsion polymer, preferably about 20 weight percent or more of the total monomer used to produce the acid swellable emulsion polymer, and most preferably about 30 weight percent or more of the total monomer used to produce acid swellable emulsion polymer. The maximum weight of the cationic ethylenically unsaturated monomer can be about 80 weight percent or less of the total monomer used to produce the acid swellable emulsion polymer, preferably about 70 weight percent or less of the total monomer used to produce the acid swellable emulsion polymer, and most preferably about 60 weight percent or less of the total monomer used to produce acid swellable emulsion polymer.

The hydrophobic ethylenically unsaturated monomers suitable for use with the acid swellable copolymer emulsions are the same as those used with the alkali swellable copolymer emulsions. The minimum amount of hydrophobic ethylenically unsaturated monomer required is an amount effective to form an acid swellable emulsion polymer, and may depend on the hydrophobicity of the monomer. That is, the higher the hydrophobicity the less monomer would be required to form an emulsion. The minimum amount of the hydrophobic ethylenically unsaturated monomer effective to form an emulsion can be about 10 weight percent or more of the total monomer used to produce the acid swellable emulsion polymer, preferably about 25 weight percent or more of the total monomer used to produce the acid swellable emulsion polymer, and most preferably about 40 weight percent or more of the total monomer used to produce the acid swellable emulsion polymer. The maximum amount of the hydrophobic ethylenically unsaturated monomer can be about 95 weight percent or less of the total monomer used to produce the acid swellable emulsion polymer, preferably about 90 weight percent or less of the total monomer used to produce the acid swellable emulsion polymer, and most preferably about 80 weight percent or less of the total monomer produce the acid swellable emulsion polymer.

The associative monomers suitable for use with the acid swellable copolymer emulsions are the same as those used with the alkali swellable copolymer emulsions. Use of the associative monomer is optional. When used, the minimum amount of the associative monomer can be about 0.1 weight percent or more of the total monomer used to produce the acid swellable emulsion polymer, preferably about 1 weight percent or more of the total monomer used to produce the acid swellable emulsion polymer, and most preferably about 2 weight percent or more of the total monomer used to produce the acid swellable emulsion polymer. The maximum amount of the associative monomer can be about 30 weight percent or less of the total monomer used to produce the acid swellable emulsion polymer, preferably about 25 weight percent or less of the total monomer used to produce the acid swellable emulsion polymer, and most preferably about 20 weight percent or less of the total used to produce the acid swellable emulsion polymer.

Further by way of example, dried rheology modifier compositions that contain an acid swellable emulsion polymer, and neutralized to a pH of 4-5 with glycolic acid, may have a water solubility of at least 0.1 weight percent at 25°C. In another embodiment, the dried rheology modifier compositions that contain an acid swellable emulsion polymer, and neutralized to a pH of 4-5 with glycolic acid, may have a water solubility of at least 0.5 weight percent at 25°C. In another embodiment, the dried rheology modifier compositions that contain an acid swellable emulsion polymer, and neutralized to a pH of 4-5 with glycolic acid, may have a water solubility of at least 1 weight percent at 25°C. In another embodiment, the dried rheology modifier compositions that contain an acid swellable emulsion polymer, and neutralized to a pH of 4-5 with glycolic acid, may have a water solubility of at least 2 weight percent at 25°C.

It is to be understood that the foregoing is for example purposes only, that many other organic and inorganic acids are known to be useful in neutralizing acid swellable emulsion polymers, and that the utility of rheology modifier compositions of the present invention comprising the acid swellable emulsion polymers is not limited to formulations having pH of 4-5.

### Emulsion Polymerization

The acid swellable and alkali swellable emulsion polymers are produced using conventional free radical emulsion polymerization. The polymerization is usually carried out in the absence of oxygen and in an inert atmosphere such as nitrogen. The continuous phase is typically water. However, minor amounts of a hydrocarbon or organic solvent can be used. In a free radical emulsion polymerization, free radical initiators that generate a free radical during the polymerization process are utilized. As used herein, the initiating system is any free radical initiating system. In an embodiment, the initiating system is soluble in water to at least 0.1 weight percent at 25°C. Suitable initiators include, but are not limited to, peroxides, azo initiators as well as redox systems, such as tert-butyl hydroperoxide and erythorbic acid, and metal ion based initiating systems. Initiators may also include both inorganic and organic peroxides. In an embodiment, the inorganic peroxides, such as sodium persulfate, potassium persulfate and ammonium persulfate, are preferred. In a further embodiment, the metal ion based initiating systems including Fe and hydrogen peroxide, as well as Fe in combination with other peroxides, are preferred. Azo initiators, especially water soluble azo initiators, may also be used. Water soluble azo initiators include, but are not limited to, 2,2'-Azobis[2-(2-imidazolin-2-yl)propane]dihydrochloride, 2,2'-Azobis[2-(2-imidazolin-2-yl)propane]disulfate dihydrate, 2,2'-Azobis(2-methylpropionamidine)dihydrochloride, 2,2'-Azobis[N-(2-carboxyethyl)-2-methylpropionamidine]hydrate, 2,2'-Azobis{2-[1-(2-hydroxyethyl)-2-imidazolin-2-yl]propane}dihydrochloride, 2,2'-Azobis[2-(2-imidazolin-2-yl)propane], 2,2'-Azobis(1-imino-1-pyrrolidino-2-ethylpropane)dihydrochloride, 2,2'-Azobis{2-methyl-N-[1,1-bis(hydroxymethyl)-2-hydroxyethl]propionamide}, 2,2'-Azobis[2-methyl-N-(2-hydroxyethyl)propionamide] and others. In an embodiment, chain transfer agents and crosslinking agents may be added during the polymerization process. Suitable chain transfer agents include, but are not limited to, mercaptans, such as, for example, dodecylmercaptan, methyl mercaptopropionate, and 3-mercaptopropionic acid, 2-mercaptoethanol, combinations thereof and the like.

Those skilled in the art will recognize that cross-linking agents can be used to affect the rheology flow curve of the rheology modifier, i.e., the viscosity of formulations containing the rheology modifier composition over a range of shear rates. These cross linking agents may also be yield to deliver yield stress properties to formulations containing the rheology modifier compositions of the present invention.

Suitable crosslinking agents include, but are not limited to, polyethylenically unsaturated copolymerizable monomers that typically have 2 or more double bonds which are effective for crosslinking, such as, for example, include di(meth)acrylate compounds such as ethylene glycol di(meth)acrylate, polyethylene glycol di(meth)acrylate, triethylene glycol di(meth)acrylate, 1 ,3-butylene glycol di(meth)acrylate, 1 ,6-butylene glycol di(meth)acrylate, 1 ,6-hexanediol di(meth)acrylate, neopentyl glycol di(meth)acrylate, 1 ,9-nonanediol di(meth)acrylate, 2,2'-bis(4-(acryloxy-propyloxyphenyl)propane, and 2,2'-bis(4-(acryloxydiethoxy-phenyl)propane; tri(meth)acrylate compounds such as, trimethylolpropane tri(meth)acrylate, trimethylolethane tri(meth)acrylate, and tetramethylolmethane tri(meth)acrylate; tetra(meth)acrylate compounds such as ditrimethylolpropane tetra(meth)acrylate, tetramethylolmethane tetra(meth)acrylate, and pentaerythritol tetra(meth)acrylate; hexa(meth)acrylate compounds such as dipentaerythritol hexa(meth)acrylate; allyl compounds such as allyl (meth)acrylate, diallylphthalate, diallyl itaconate, diallyl fumarate, and diallyl maleate; polyallyl ethers of sucrose having from 2 to 8 allyl groups per molecule, polyallyl ethers of pentaerythritol such as pentaerythritol diallyl ether, pentaerythritol triallyl ether, and pentaerythritol tetraallyl ether, and combinations thereof; polyallyl ethers of trimethylolpropane such as trimethylolpropane diallyl ether, trimethylolpropane triallyl ether, and combinations thereof. Other suitable crosslinkers include divinyl glycol, divinyl benzene, and methylenebisacrylamide.
In another aspect, crosslinkers can be synthesized via an esterification reaction of a polyol made from ethylene oxide or propylene oxide or combinations thereof with unsaturated anhydride such as maleic anhydride, citraconic anhydride, itaconic anhydride, or an addition reaction with unsaturated isocyanate such as 3-isopropenyl-α-α- dimethylbenzene isocyanate. Mixtures of two or more of the foregoing crosslinkers can also be utilized.

In an embodiment of the invention, the monomer composition can be adjusted from the inside to the outside of the polymer particle formed during the emulsion polymerization in a manner to give the best performance properties for a particular application. The monomer concentration can be lower in hydrophilic monomer in the inside of the polymer particle and higher in hydrophilic monomer content on the outside of the polymer particle and vice versa.
The use of stabilizing surfactants in the polymerization reaction mixture during the polymerization of acid-swellable emulsion polymers is preferred where one or more of the monomers is susceptible to hydrolysis during polymerization, such as dimethylaminoethyl methacrylate or tertbutylamino ethyl methacrylate or mixtures thereof. The stabilizing surfactants help to protect these monomers from hydrolysis during the polymerization process by minimizing their contact with water. Stabilizing surfactants are optionally present in the polymerization reaction mixture during the polymerization of alkali swellable emulsion polymers., and in the polymerization of acid swellable emulsion polymers if the cationic monomer is not dimethylaminoethyl methacrylate, tertbutylamino ethyl methacrylate, or any other monomer susceptible to hydrolysis Such surfactants serve to stabilize the emulsion during the polymerization process. These stabilizing surfactants can be anionic or non-ionic for the preparation of the alkali swellable emulsion polymers, and can be cationic or non-ionic for the preparation of the acid swellable emulsion polymers. Examples of anionic surfactants are alkylbenzenesulfonic acids, sulfonated fatty acids, sulfosuccinates, fatty alcohol sulfates, alkylphenol sulfates and fatty alcohol ether sulfates. Examples of nonionic surfactants are alkylphenol ethoxylates, primary alcohol ethoxylates, fatty acid ethoxylates, alkanolamide ethoxylates, fatty amine ethoxylates, EO/PO block copolymers and alkylpolyglucosides. Examples of cationic and amphoteric surfactants used are quaternized amine alkoxylates, alkylbetaines, alkylamidobetaines and sulfobetaines. Stabilizing surfactants which have a reactive double bond can also be used. Stabilizing surfactants (see US 20140114006) which have more than one reactive double bond can also be used, and can also act as crosslinking agents.

Dried rheology modifier compositions of the present invention may comprise an anti-caking agent. Examples of anticaking agents include but are not limited to kaolin, aluminosilicates, silicon oxide, aluminum silicon oxide, calcium carbonate, magnesium carbonate, magnesium sulfate, talc, gypsum, silica and silicates, and mixtures thereof. The particle sizes of the anticaking agents are preferably in the range of from 100 nm to 10 µm. More than one anticaking agent may be used.

The dried rheology modifier compositions of the present invention can be emulsion polymers which are blended with the water soluble support polymer and then dried. The emulsion polymers are first prepared as described above and then the water soluble support polymer is added. In a preferred embodiment, the blend can be made by adding an aqueous solution of the water soluble support polymer to the emulsion polymer with suitable amount of mixing. Alternatively, the emulsion polymer can be added to aqueous solution of the water soluble support polymer. In an embodiment, a dry water soluble support polymer is added to the emulsion polymer with concurrent dilution in water. The weight percent of polymer in the emulsion polymer may be in the range of 5-50% and preferably in the range 10-30%. The solids of the aqueous solution of the water soluble support polymer may be in the range of 5-50% and preferably in the range 10-30%. The aqueous blend of the emulsion polymer and the water soluble support polymer may be in the range of 5-50% and preferably in the range 10-30% and most preferably in the range 15-25%. This blend may be dried as is or may be further diluted if necessary before drying. The preferred drying method is spray drying. However, other methods such as drum drying, tray drying, fluidized bed drying etc may be used.

### Product formulations

The present invention relates to product formulations comprising the dried rheology modifier compositions as disclosed herein, wherein the product formulations may be selected from personal care products, home care products, healthcare products, institutional and industrial care products, adhesives, coatings, agricultural and other applications, and the product formulations also contain a product active ingredient (as further explained below for certain product formulations). The present invention further relates to the use of the dried rheology modifier compositions as disclosed herein in such product formulation, wherein the product formulation may be selected from the group consisting of a personal care product formulation, a home care product formulation, a healthcare product formulation, an institutional and industrial care product formulation, an adhesive, an agricultural product formulation, an oil field fracturing product formulation, an asphalt product formulation, a paint formulation, and a water-based protective coating product formulation.

The term "home care products" as used herein includes, without being limited thereto, products employed in a domestic household for surface cleaning or maintaining sanitary conditions, such as in the kitchen and bathroom (e.g., hard surface cleaners, furniture polishes, hand and automatic dish washing formulations, toilet bowl cleaners and disinfectants), and laundry products for fabric care and cleaning (e.g., detergents, fabric conditioners, pre-treatment stain removers), and the like.

The term "health care products" as used herein includes, without being limited thereto, pharmaceuticals (controlled release pharmaceuticals), pharmacosmetics, oral care (mouth and teeth) products, such as oral suspensions, mouthwashes, toothpastes, dentifrices, and the like, and over-the-counter products and appliances (topical and transdermal), such as patches, plasters and the like, externally applied to the body, including the skin, scalp, nails and mucous membranes of humans and animals, for ameliorating a health-related or medical condition, for generally maintaining hygiene or well-being, and the like.

The term "institutional and industrial care" ("I&I") as used herein includes, without being limited thereto, products employed for surface cleaning or maintaining sanitary conditions in institutional and industrial environments, textile treatments (e.g., textile conditioners, carpet and upholstery cleaners), automobile care (e.g., hand and automatic car wash detergents, tire shines, leather conditioners, liquid car polishes, plastic polishes and conditioners), paints and coatings, and the like.

In agricultural applications, the dried rheology modifier compositions of this invention are useful in agrochemical formulations. They can provide stabilization, thickening, dispersion, or suspension properties to the agrochemical formulations due to their rheological properties. One particularly useful agrochemical formulation is a suspension concentrate (SC). Another particularly useful agrochemical formulation is a solid formulation including wettable powder (WP), water dispersible granule (WDG), and water soluble granule (WSG). When an agrochemical formulation comprising the dried rheology modifier compositions of the present invention is diluted into water, the dried rheology modifier compositions of the present invention can stabilize or suspend agrochemicals in diluted aqueous systems.

In oil field applications, the dried rheology modifier compositions of this invention can be used in formulations used in fracturing operations. In some applications, it is desired to use liquid compositions with viscoelastic properties. Such compositions, for instance, may be used to stimulate oil wells wherein impeded flow paths lead to an insufficient hydrocarbon production, a technique known as (hydraulic) fracturing and the specialized fluids used in said technique are referred to as fracturing fluids. For such a fracturing process, the compositions are typically injected via the wellbore into the formation at sufficient pressures to create fractures in the formation rocks, thus creating channels through which the hydrocarbons may more readily flow into the wellbore. In an embodiment, the fracturing fluids should impart a minimal pressure drop in the pipe within the wellbore during placement and have an adequate viscosity to carry proppant (sand) material that prevents the fracture from closing. Moreover, the fracturing fluids should have a minimal leak-off rate to avoid fluid migration into the formation rocks so that, notably, the fracture can be created and propagated and should degrade so as not to leave residual material that may prevent accurate hydrocarbons to flow into the wellbore.

Other formulations in which the rheology modifiers of the present invention can be used include adhesives, asphalt emulsions, paints and coatings, superabsorbents and other industrial applications.

In an embodiment, the dried rheology modifiers compositions of this invention may be added to these formulations at least about 0.1% modifier by weight of the formulation, more preferably at least about 0.5% modifier by weight of the formulation and most preferably at least about 1.0% modifier by weight of the formulation. The dried rheology modifiers compositions of this invention may be added to these formulations at most about 20% modifier by weight of the formulation, more preferably at most about 15% modifier by weight of the formulation and most preferably at most about 10% modifier by weight of the formulation.

The rheology modifiers of this invention can be used in aqueous protective coating compositions. These rheology modifiers increase and maintain the viscosity at required levels under specific processing conditions and end-use situations. In particular, the rheology modifiers of this invention are useful in all kinds of coatings such as decorative and protective coatings. The rheology modifiers of this invention can be used as rheology modifiers for water-based protective coating compositions. Water-based protective coating compositions are commonly known as latex paints or dispersion paints and have been known for a considerable number of years. The adjustment of the rheology properties of such an aqueous protective coating composition is challenging, since the coating composition must provide good leveling and excellent sag resistance, yet also have a viscosity which is neither too low nor too high in order to allow an easy application.

The polymers of this invention can be used in paper coating applications. A paper coating formulation imparts certain qualities to the paper, including weight, surface gloss, smoothness or reduced ink absorbency. A uniform coating on paper contributes to an enhanced printing surface and properties such as coverage, smoothness, and gloss may be improved. Paper and board grades are sometimes coated to improve the printability, visual properties, or functionality of the sheet. The properties and printability of coated papers are affected by the base sheets (fiber types, sheet formation, internal sizing, and base weight), coating materials (pigment types, binder types, rheology modifiers, water-retention aids, lubricants, defoamers, etc.), coating formulations (ratios of coating components, solids and pH's), coating process (coating application types and speed), coat weights, drying conditions (dryer types, drying temperature, drying time, and final moisture level), etc.

Paper coating formulas typically contain three main categories of ingredients: pigments, binders and additives. Pigments improve printing and optical properties of the sheet, binders adhere the pigment particles to each other and to the sheet, and additives either assist in the coating process or enhance sheet properties. Among the key additives used in paper coating formulations are rheology modifiers. Rheology modifiers are used to achieve desired rheological properties as well as improved runnability during the coating process.

The dried rheology modifier compositions of the present invention, especially those that contain the alkali swellable polymers, uncoil when neutralized in the coating formulation, which increases the viscosity. This viscosity increase helps control pick up rates on applicator rolls, affects flow properties during metering processes and changes immobilization and leveling properties after the metering step in the coating process. The ratio of the hydrophilic to hydrophobic monomers in the polymer affects water retention properties and the degree of interaction with the binder. The molecular weight of the polymer and its branching affect both the low shear and high shear viscosity profile.

The dried rheology modifier compositions which contain a hydrophobically modified alkali swellable polymer are also useful in paper coating applications. Such products are highly effective in lightweight coatings (LWC). These products are used in high solids carbonate coatings, which require water retention with minimal high shear viscosity development.

The term "personal care products" as used herein includes without limitation cosmetics, toiletries, cosmeceuticals, beauty aids, insect repellents, personal hygiene and cleansing products applied to the body, including the skin, hair, scalp, and nails of humans and animals. The personal care applications include, but are not limited to, formulations for hair styling gels, skin creams, sun tan lotions, sunscreens, moisturizers, tooth pastes, medical and first aid ointments, cosmetic ointments, suppositories, cleansers, lipstick, mascara, hair dye, cream rinse, shampoos, body soap and deodorants, hair care and styling formulations, shaving preparations, depilatories and hand sanitizers, including alcohol based hand sanitizers.

Suitable personal care applications also include formulations for use on the skin, eyelashes or eyebrows, including, without limitation, cosmetic compositions such as mascara, facial foundations, eyeliners, lipsticks, and color products; skin care compositions such as moisturizing lotions and creams, skin treatment products, skin protection products in the form of an emulsion, liquid, stick, or a gel; sun care compositions such as sunscreens, sunscreen emulsions, lotions, creams, sunscreen emulsion sprays, liquid/alcohol sunscreen sprays, sunscreen aqueous gels, broad spectrum sunscreens with UVA and UVB actives, sunscreens with organic and inorganic actives, sunscreens with combinations of organic and inorganic actives, suntan products, self-tanning products, and after sun products etc. Particularly suitable compositions are personal care emulsions, more particularly suitable are sun care compositions such as sunscreen emulsions and sunscreen emulsion sprays. The personal care composition may be in any form, including without limitation in sprays, emulsions, lotions, gels, liquids, sticks, waxes, pastes, powders, and creams.

The personal care compositions may also include other optional components commonly used in the industry, and these will vary greatly depending upon the type of composition and the functionality and properties desired. Without limitation, these components include thickeners, suspending agents, emulsifiers, UV filters, sunscreen actives, humectants, moisturizers, emollients, oils, waxes, solvents, chelating agents, vitamins, antioxidants, botanical extracts, silicones, neutralizing agents, preservatives, fragrances, dyes, pigments, conditioners, polymers, antiperspirant active ingredients, antiacne agents, anti-dandruff actives, surfactants, exfoliants, depilatory active ingredients, film formers, propellants, tanning accelerator, hair fixatives and colors. The polymers of the present invention are compatible with most other components used in conventional personal care compositions. For example, sunscreen compositions may contain at least one component selected from the group comprising organic UV filters, inorganic UV actives, UVA and/or UVB suncreen actives, octinoxate, octisalate, oxybenzone, homosalate, octocrylene, avobenzene, titanium dioxide, starch, conditioning agents, emulsifiers, other rheology modifiers and thickeners, neutralizers, emollients, solvents, film formers, moisturizers, antioxidants, vitamins, chelating agents, preservatives, fragrances, and zinc oxide. Skin care and cosmetic compositions may contain at least one component selected from the group consisting of vitamins, anti-aging agents, moisturizers, emollients, emulsifiers, surfactants, preservatives, pigments, dyes, colors and insect repellents.

When used in personal care formulations, such as hair care and styling formulations, for example styling gels, optional additional ingredients can be added to provide a variety of further additional properties. Various other additives, such as active and functional ingredients, may be included in the personal care formulation as defined herein. These include, but are not limited to, emollients, humectants, thickening agents, electrolytes and salts surfactants, UV light inhibitors, fixative polymers preservatives pigments dyes, colorants, alpha hydroxy acids, aesthetic enhancers such as starch perfumes and fragrances, film formers (water proofing agents) antiseptics, antifungal, antimicrobial and other medicaments and solvents. Additionally, conditioning agents can be used in combination with the polymers of this invention, for example, cationic guar gum, cationic hydroxyethyl cellulose, cationic synthetic polymers and cationic fatty amine derivatives. These blended materials help to provide more substantivity and effective conditioning properties in hair. The electrolytes and salts are particularly useful in boosting the viscosity of the shampoo and improving its suspending properties.
In one embodiment, the dried rheology modifiers of this invention contain an alkali swellable emulsion polymer. In one embodiment, the dried rheology modifiers of this invention contain an alkali swellable emulsion polymer that has to be neutralized with an alkali to the pH of 6-6.5 and above to be activated. However, it is desirable to have some formulations, especially personal care formulations, in the pH range 4 to 5.5. For such formulations, the dried rheology modifier composition containing the alkali swellable polymers can be mixed into an aqueous formulation containing surfactants, activated by neutralization to a pH of about 6.5 or higher and subsequently acidified in the presence of a surfactant to lower the formulation pH to 3-6.5, preferably to 4 - 5.5 in accordance with the back acid titration process described in US patent numbers 4,529,773, 6,635,702 and 6,897,253.

Some non-limiting examples of polymers that can used in personal care formulations in conjunction with the dried rheology modifier compositions of this invention are polyoxythylenated vinyl acetate/crotonic acid copolymers, vinyl acetate crotonic acid (90/10) copolymers, vinyl acetate/crotonic acid/vinyl neodecanoate terpolymers, N-octylacrylamide/methylacrylate/hydroxypropyl methacrylate/acrylic acid/tert-butylaminoethyl methacrylate copolymers, and methyl vinyl ether/maleic anhydride (50/50) copolymers monoesterified with butanol or ethanol, acrylic acid/ethyl acrylate/N-tert-butyl-acrylamide terpolymers, and poly (methacrylic acid/acrylamidomethyl propane sulfonic acid), acrylates copolymer, octylacrylamide/acrylates/butylaminoethyl methacrylate copolymer, acrylates/octylacrylamide copolymer, VA/crotonates/vinyl Neodeanoate copolymer, poly(N-vinyl acetamide), poly(N-vinyl formamide), corn starch modified, sodium polystyrene sulfonate, polyquaterniums such as polyquaternium-4, polyquaternium-7, polyquaternium-10, polyquaternium-11, polyquarternium-16, polyquaternium-28, polyquaternium-29, polyquaternium-46, polyether-1, polyurethanes, VA/acrylates/lauryl methacrylate copolymer, adipic acid/dimethylaminohydroxypropyl diethylene AMP/acrylates copolymer, methacrylol ethyl betaine/acrylates copolymer, PVP/dimethylaminoethylmethacrylate copolymer, PVP/DMAPA acrylates copolymer, PVP/vinylcaprolactam/DMAPA acrylates copolymer, vinyl caprolactam/PVP/dimethylaminoethyl methacrylate copolymer, VA/butyl maleate/isobomyl acrylate copolymer, VA/crotonates copolymer, acrylate/acrylamide copolymer, VA/crotonates/vinyl propionate copolymer, vinylpyrrolidone/vinyl acetate/vinyl propionate terpolymers, VA/crotonates, cationic and amphoteric guar, polyvinylpyrrolidone (PVP), polyvinylpyrrolidone/vinyl acetate copolymer, PVP acrylates copolymer, vinyl acetate/crotonic acid/vinyl proprionate, acrylates/acrylamide, acrylates/octylacrylamide, acrylates/hydroxyacrylates copolymer, and alkyl esters of polyvinylmethylether/maleic anhydride, diglycol/cyclohexanedimethanol/isophthalates/sulfoisophthalates copolymer, vinyl acetate/butyl maleate and isobornyl acrylate copolymer, vinylcaprolactam/PVP/dimethylaminoethyl methacrylate, vinyl acetate/alkylmaleate half ester/N-substituted acrylamide terpolymers, vinyl caprolactam/vinylpyrrolidone/ methacryloamidopropyl trimethylammonium chloride terpolymer methacrylates/acrylates copolymer/amine salt, polyvinylcaprolactam, polyurethanes, hydroxypropyl guar, hydroxypropyl guar hydroxypropyl trimmonium chloride, poly (methacrylic acid/acrylamidomethyl propane sulfonic acid, poylurethane/acrylate copolymers and hydroxypropyl trimmonium chloride guar, particularly acrylates copolymer, octylacrylamide/acrylates/butylaminoethyl methacrylate copolymer, acrylates/octylacrylamide copolymer, VA/crotonates/vinyl Neodecanoate copolymer, poly(N-vinyl acetamide), poly(N-vinyl formamide), polyurethane, corn starch modified, sodium polystyrene sulfonate, polyquaternium-4, polyquarternium-10, and polyurethane/acrylates copolymer.

Suitable cationic polymers that may be used in formulations comprising the dried rheology modifier compositions of the present invention are those best known with their CTFA category name Polyquaternium. Some examples of this class of polymer are Polyquaternium 6, Polyquaternium 7, Polyquaternium 10, Polyquaternium 11, Polyquaternium 16, Polyquaternium 22 and Polyquaternium 28, Polyquaternium 4, Polyquaternium 37, Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

Naturally derived cellulose type polymers known as Polymer JR® type from Amerchol, Polyquaternium 10 or cationic guar gum known with trade name Jaguar® from Rhone-Poulenc, and Guar hydroxypropyl trimonium chloride, chitosan and chitin can also be included in the personal care formulations as cationic natural polymers in formulations comprising the dried rheology modifier compositions of the present invention.

The dried rheology modifiers of this invention may be used in personal care compositions that may also include a cosmetically acceptable ingredient. The ingredient can be an emollient, fragrance, exfoliant, medicament, whitening agent, acne treatment agent, a preservative, vitamins, proteins, a cleanser or conditioning agent.

Examples of cleansers suitable for use in compositions of the present invention include, but are not limited to, are sodium lauryl sulfate (SLS), sodium laureth sulfate (SLES), ammonium lauryl ether sulfate (ALES), alkanolamides, alkylaryl sulfonates, alkylaryl sulfonic acids, alky1benzenes, a e acetates, amine oxides, amines, sulfonated amines and amides, betaines, block polymers, carboxylated alcohol or alkylphenol ethoxylates, diphenyl sulfonate derivatives, ethoxylated alcohols, ethoxylated alkylphenols, ethoxylated amines and/or amides, ethoxylated fatty acids, ethoxylated fatty esters and oils, fatty esters (other than glycol, glycerol, etc.), fluorocarbon-based surfactants, glycerol esters, glycol esters, heterocyclics, imidazolines and imidazoline derivatives, isethionates, lanolin-based derivatives, lecithin and lecithin derivatives, lignin and lignin derivatives, methyl esters, monoglycerides and derivatives, olefin sulfonates, phosphate esters, phosphorous organic derivatives, polymeric (polysaccharides, acrylic acid, acrylamide), propoxylated and ethoxylated fatty acids, propoxylated and ethoxylated fatty alcohols, propoxylated and ethoxylated alkyl phenols, protein-based surfactants, quaternary surfactants, sarcosine derivatives, silicone-based surfactants, soaps, sorbitan derivative, sucrose and glucose esters and derivatives, sulfates and sulfonates of oils and fatty acids, sulfates and sulfonates ethoxylated alkyl phenols, sulfates of alcohols, sulfates of ethoxylated alcohols, sulfates of fatty esters, sulfonates of benzene, cumene, toluene and xylene, sulfonates of condensed naphthalenes, sulfonates of dodecyl and tridecyl benzenes, sulfonates of naphthalene and alkyl naphthalene, sulfonates of petroleum, sulfosuccinamates, sulfosuccinates and derivatives.

In an embodiment of this invention, particularly where the formulation is a shampoo or a cleaning formulation such as a body wash, the formulation further comprises a sulfate free surfactant. Examples of sulfate free surfactants include, but are not limited to, ethoxylated alkylphenols, ethoxylated amines and/or amides, ethoxylated fatty acids, ethoxylated fatty esters and oils, fatty esters (other than glycol, glycerol, etc.), fluorocarbon-based surfactants, glycerol esters, glycol esters, heterocyclics, imidazolines and imidazoline derivatives, isethionates, lanolin-based derivatives, lecithin and lecithin derivatives, lignin and lignin derivatives, methyl esters, monoglycerides and derivatives, phosphate esters, phosphorous organic derivatives, polymeric (polysaccharides, acrylic acid, acrylamide), propoxylated and ethoxylated fatty acids, propoxylated and ethoxylated fatty alcohols, propoxylated and ethoxylated alkyl phenols, protein-based surfactants, quaternary surfactants, sarcosine derivatives, siliconebased surfactants, alpha-olefin sulfonate, alkylaryl sulfonates, sulfonates of oils and fatty acids, sulfonates of ethoxylated alkyl phenols, sulfonates of benzene, cumene, toluene and xylene, sulfonates of condensed naphthalenes, sulfonates of dodecyl and tridecyl benzenes, sulfonates of naphthalene and alkyl naphthalene, sulfonates of petroleum and derivatives thereof. In an embodiment of the invention, the sulfate free surfactants are sulfonates or ethoxylates.

In another embodiment the formulation contains sulfated surfactants. Some non-limiting examples of sulfated surfactants are sodium lauryl sulfate (SLS), sodium laureth sulfate (SLES), alkanolamides, alkylaryl sulfonic acids, sulfates of oils and fatty acids, sulfates of ethoxylated alkyl phenols, sulfates of alcohols, sulfates of ethoxylated alcohols, sulfates of fatty esters, sulfosuccinamates, sulfosuccinates and derivatives thereof.

In other embodiments of the invention, the personal care formulation comprising the inventive rheology modifier is a hair fixative or styling formulation, such as a hair gel, mousse, spray, pomade, wax, or styling lotion. Surprisingly, it has been found that rheology modifiers of the present invention can be formulated into such hair fixative and styling formulations, to produce not only desired rheology modification, but also particle suspension properties and hair holding properties; dred rheology modifiers of the present invention that have been heat treated are particularly suitable for such formulations. When the rheology modifiers of the present invention are used in such formulations, it is possible to reduce or even eliminate other additives that have traditionally been used to provide these functions.

In addition to the polymer(s) of this invention, personal care compositions may optionally include other ingredients. Some non-limiting examples of these ingredients include, but are not limited to, conditioning agents such as silicone oils, either volatile or non-volatile, natural and synthetic oils. Suitable silicone oils that can be added to the compositions include dimethicone, dimethiconol, polydimethylsiloxane, silicone oils with various DC fluid ranges from Dow Corning. Suitable natural oils, such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate can also be used. Some examples of non-ionic conditioning agents are polyols such as glycerin, glycol and derivatives, polyethyleneglycols, which may be known by the trade names Carbowax® PEG from Union Carbide and Polyox® WSR range from Amerchol, polyglycerin, polyethyleneglycol mono- or di- fatty acid esters.

Preservatives can be used in personal care formulations of the present invention to provide long term shelf stability. These can be selected from among methylparaben, propylparaben, butylparaben, DMDM hydantoin, imidazolidinyl urea, gluteraldehyde, phenoxyethanol, benzalkonium chloride, methane ammonium chloride, benzethonium chloride, benzyl alcohol, chlorobenzyl alcohol, methylchloroisothiazolinone, methylisothiazolinone, sodium benzoate, chloracetamide, triclosan, iodopropynyl butylcarbamate, sodium pyrithione, and zinc pyrithione.
The dried rheology modifier compositions of this invention can also be used in liquid detergent compositions that include one or more surfactants, such as those selected from anionic, nonionic, cationic, amphoteric, and zwitterionic surfactants. In an embodiment, the preferred surfactants are suitable for use in isotropic liquid detergent compositions and are mixtures of anionic and nonionic surfactants although it is to be understood that any surfactant may be used alone or in combination with any other surfactant or surfactants. These liquid detergent systems as well as the surfactants used in them are described in US 6,462,013.

Liquid detergent compositions comprising dried rheology modifier compositions of this invention may also be used in liquid detergent compositions and may further optionally comprise at least one additive. Suitable additives may include, for example, builders, dispersants, polymers, ion exchangers, alkalis, anticorrosion materials, antiredeposition materials, antistatic agents, optical brighteners, perfumes, fragrances, dyes, fillers, oils, chelating agents, enzymes, fabric whiteners, brighteners, sudsing control agents, solvents, hydrotropes, bleaching agents, bleach precursors, buffering agents, soil removal agents, soil release agents, fabric softening agents, and opacifiers. In general, such additives and their amounts are known to those skilled in the art.

### EXAMPLES

The following examples are intended to illustrate various embodiments of the invention, and are not intended to limit the scope of the invention which is defined in the claims appended hereto.

### Drying

The drying step in the following examples was accomplished by spray drying on a Mobile Minor spray dryer available from GEA Process Engineering Inc. at a flow rate between 8 and 12 on a Masterflex 7575-00 pump and an outlet temperature ranging from 80 to 95°C. However, any conventional spray dryer can be used, or other conventional drying means.

### Particle size measurement

Particle sizes as reported in the Examples were determined by the following procedure:
- 0.1 g of emulsion polymer was diluted to 100 mL with room temperature water and agitated to mix.
- An appropriate amount of the diluted emulsion was dispensed into a standard 4.5 mL polystyrene cuvette.
- Particle size was analyzed with Malvern Zetasizer Nano S Red Badge (model ZEN1600) instrument:
- Measurements were made under manual measurement setting using factory defaults, such that the instrument ran optimization processes before taking measurements, and there was nothing manually changed from the default factory parameter settings.
- Three measurements were taken with 15 runs each.
- Z-average particle size was reported.

### Molecular weight determination

Weight average molecular weight of the starches of Examples 1-7 were determined by the following procedure:
Starch samples were prepared at 2.00 mg/ml in 0.03M NaCl in dimethylsulfoxide (DMSO). The starch sample solutions were heated at 100°C for 60 minutes and were clear after heating. The solutions were filtered using a 0.45 micron filter. The molecular weight was measured using gel permeation chromatography with multi-angle light scattering ("GPC/MALS") detection as follows:

| | |
|---|---|
| Column | Phenogel Linear 2 30cm x 7.8mm |
| Temperature | 60°C |
| Solvent | 0.03M NaCl in DMSO |
| Flow Rate | 0.60 ml/min |
| Detection | Wyatt Heleos 18 angle MALS and Optilab Rex Refractive Index |

### Viscosity measurements

In these examples, the procedures used to measure viscosity varied slightly in accordance with the type of sample being evaluated, as follows:
- When measuring the viscosity of rheology modifiers in water, viscosities were measured at 25°C using a RVDV-1 Brookfield viscometer (Brookfield Engineering Middleboro, MA) using the supplied RV2 spindle at 10 rpm. Samples were allowed to equilibrate for 1 minute before a reading was taken.
- When measuring the viscosity of rheology modifiers in surfactant mixtures, viscosities were measured at 25°C using a RVDV-1 Brookfield viscometer (Brookfield Engineering Middleboro, MA) using the supplied RV5 spindle at 10 rpm. Samples were allowed to equilibrate for 1 minute before a reading was taken.
- When measuring the viscosity of hair gel formulations viscosities were measured at 25°C using a RVDV-1 Brookfield viscometer (Brookfield Engineering Middleboro, MA) fitted with a helipath stand using the supplied TC spindle at 10 rpm. Samples were allowed to equilibrate before a reading was taken.

### Turbidity measurements

Turbidity was measured by placing samples in a HACH tube (glass tubes designed for the specific instrument). The samples were centrifuged at 250 rpm for 10 minutes. The samples were then measured on a HACH Turbidimeter (model number 2100N) and reported in NTU. Turbidity was measured at room temperature (approximately 23°C).

### Example 1: Polysaccharide present during polymerization reaction

1348.6 grams of water and 100.1 g of 80.9% PR1004B potato starch with a weight average molecular weight of 862,000 (from Avebe) were charged into a reactor and heated to 82°C. An overhead nitrogen sparge was started upon initial heating of the water. The starch was cooked for 30 minutes at 82°C. A monomer solution containing 151.6 grams ethyl acrylate, 87.7 grams methacrylic acid, and 0.954 grams trimethylolpropane triacrylate was subsequently added to the reactor over a period of 90 minutes. An initiator solution containing 1 gram of ammonium persulfate and 100.5 grams water was added to the reactor at the same time as the monomer solutions over a period of 90 minutes. The reaction product was held at 82°C for an additional 120 minutes. The product was cooled to room temperature. 14.25 grams of 10% sodium hydroxide was added to the reactor over 30 minutes and mixed for 30 minutes. The final product was a homogeneous, white emulsion with a total solids content of around 17.5 weight% and a pH of 5.2 and an average particle size of 525 nm. This reaction product was then spray dried to produce a non-tacky white powder with an average particle size of around 10-20 microns.

### Example 2: Polysaccharide present during polymerization reaction

1185 grams of water and 84.7 g of 88.4% PR1303A potato starch (weight average molecular weight of 2,259,000) (from Avebe) were charged into a reactor and heated to 82°C. An overhead nitrogen sparge was started upon initial heating of the water. The starch solution was cooked for 30 minutes at 82°C. A monomer solution containing 95.7 grams ethyl acrylate, 122.3 grams methacrylic acid, and 0.895 grams trimethylolpropane triacrylate was subsequently added to the reactor over a period of 90 minutes. An initiator solution containing 0.94 grams ammonium persulfate and 134.7 grams water was added to the reactor at the same time as the monomer solution over a period of 90 minutes. The reaction product was held at 82°C for an additional 120 minutes. The heat was turned off and nitrogen was blown over the sample during cooling to remove ethyl acrylate, during which time an additional 39 grams of water was added to the reactor. The final product was a homogeneous, white emulsion with a total solids content of around 17.5 weight% and a pH of 2.4 and an average particle size of 380 nm. This reaction product was then spray dried to produce a non-tacky white powder.

### Example 3: Polysaccharide present during polymerization reaction

1400 grams of water and 56.9 g of 80.9% 1401B potato starch with a weight average molecular weight of 1,288,000 (from Avebe) were charged into a reactor and heated to 82°C. An overhead nitrogen sparge was started upon initial heating of the water. The starch was cooked for 30 minutes at 82°C. A monomer solution containing 95.5 grams ethyl acrylate, 55.3 grams methacrylic acid, and 0.6 grams trimethylolpropane triacrylate was subsequently added to the reactor over a period of 90 minutes. An initiator solution containing 0.63 gram of ammonium persulfate and 100.5 grams water was added to the reactor at the same time as the monomer solutions over a period of 90 minutes. The reaction product was held at 82°C for an additional 120 minutes. The product was cooled to room temperature. 12.8 grams of 10% sodium hydroxide was added to the reactor over 30 minutes and mixed for 30 minutes. The final product was a homogeneous, white emulsion with a total solids content of around 11.5 weight %, an average particle size of 401 nm, and a pH of 5. This reaction product was then spray dried to produce a non-tacky white powder.

### Example 4: Polysaccharide present during polymerization reaction

628.5 grams of water and 135.1 g of 80.9% PR1004B potato starch (weight average molecular weight 862,000, from Avebe) were charged into a reactor and heated to 82°C. An overhead nitrogen sparge was started upon initial heating of the water. The starch was cooked for 30 minutes at 82°C. A monomer solution containing 45.4 grams ethyl acrylate, 26.3 grams methacrylic acid was subsequently added to the reactor over a period of 20 minutes. An initiator solution containing 0.3 gram of ammonium persulfate and 60 grams water was added to the reactor at the same time as the monomer solutions over a period of 20 minutes. At the end of the feeds the reaction product was diluted by slowly adding 225 grams of water. The reaction product was held at 82°C for an additional 120 minutes. The product was cooled to room temperature. 4.3 grams of 10% sodium hydroxide was added to the reactor over 30 minutes and mixed for 30 minutes. The final product was a homogeneous, white emulsion with a total solids content of around 14.8 weight % and a pH of 5.2. This reaction product was then spray dried to produce a non-tacky white powder.

### Example 5: Polysaccharide present during polymerization reaction

644 grams of water and 112.3 g of 80.9% PR1004B potato starch (weight average molecular weight 862,000, from Avebe) were charged into a reactor and heated to 82°C. An overhead nitrogen sparge was started upon initial heating of the water. The starch was cooked for 30 minutes at 82°C. A monomer solution containing 56.6 grams ethyl acrylate, 32.7 grams methacrylic acid was subsequently added to the reactor over a period of 30 minutes. An initiator solution containing 0.3 gram of ammonium persulfate and 60 grams water was added to the reactor at the same time as the monomer solutions over a period of 30 minutes. At the end of the feeds the reaction product was diluted by slowly adding 225 grams of water. The reaction product was held at 82°C for an additional 120 minutes. The product was cooled to room temperature. 5.3 grams of 10% sodium hydroxide was added to the reactor over 30 minutes and mixed for 30 minutes. The final product was a homogeneous, white emulsion with a total solids content of around 15.5 weight% and a pH of 5. This reaction product was then spray dried to produce a non-tacky white powder.

### Example 6: Polysaccharide present during polymerization reaction

1400 grams of water and 170.8 g of 88.4 % 1401B potato starch (weight average molecular weight 1,288,000, from Avebe) were charged into a reactor and heated to 82°C. An overhead nitrogen sparge was started upon initial heating of the water. The starch was cooked for 30 minutes at 82°C. A monomer solution containing 31.8 grams ethyl acrylate, 18.4 grams methacrylic acid, and 0.2 grams trimethylolpropane triacrylate was subsequently added to the reactor over a period of 90 minutes. An initiator solution containing 0.22 gram of ammonium persulfate and 100.5 grams water was added to the reactor at the same time as the monomer solutions over a period of 90 minutes. The reaction product was held at 82°C for an additional 120 minutes. The final product was a homogeneous, white emulsion with a total solids content of around 11.7 weight %, an average particle size of 235 nm, and a pH of 2.8. This reaction product was then spray dried to produce a non-tacky white powder with an average particle size of around 10-20 microns.

### Example 7: Polysaccharide present during polymerization reaction

741 grams of water and 55 g of 80.9% PR1004B potato starch (weight average molecular weight 862,000, from Avebe) were charged into a reactor and heated to 82°C. An overhead nitrogen sparge was started upon initial heating of the water. The starch was cooked for 30 minutes at 82°C. A monomer solution containing 83.3 grams ethyl acrylate, 48.2 grams methacrylic acid, and 0.545 grams trimethylolpropane triacrylate was subsequently added to the reactor over a period of 90 minutes. An initiator solution containing 0.554 gram of ammonium persulfate and 55 grams water was added to the reactor at the same time as the monomer solutions over a period of 90 minutes. The reaction product was held at 82°C for an additional 120 minutes. The product was cooled to room temperature. 7.9 grams of 10% sodium hydroxide was added to the reactor over 30 minutes and mixed for 30 minutes. The final product was a homogeneous, white emulsion and was then spray dried to produce a non-tacky white powder.

### Example 8: Typical procedures for heat treatment

Several of the spray dried samples from Examples 1-6 above were treated by heating 20 grams of the powder in a vented oven at temperatures between 100 and 140° C. for a period of 15 to 180 minutes. The samples before and after heat treatment were formulated into hair gels and the viscosities of these hair gels were measured.

### Example 9: Hair gel formulations

### Typical procedure:

Hair gel samples were formulated by mixing Solutions A and B below, in which the amounts are stated as parts per 100 grams of formulation:

**Solution A**

| | |
|---|---|
| Polyvinyl pyrrolidone K-90 | 3.0 |
| Triethanolamine (to neutralize to pH 7) | ∼0.4-0.5 grams per gram of polymer |
| DI water | 46 |

**Solution B**

| | |
|---|---|
| Dried rheology modifier composition of invention | 1.0-2.0 |
| DI water | 47.5- 48.5 |
| Glydant® Plus preservative (Lonza) | 0.5 |

The DI water in Solution A was adjusted so that the total weight of the formulation was 100 grams.

### Example 10: Evaluation of Rheology

Quantities of the spray-dried rheology modifier powders of Example 1 were heat treated in a 130°C oven for varying times ranging from 0-75 minutes. These powders then were formulated into hair gels using 1% polymer as described in Example 9, except that the neutralization with triethanolamine was omitted for Example 10-8. Viscosities were evaluated after 24 hours using a Brookfield viscometer model DV-I Prime spindle TC at 10 rpm with helipath at room temperature.

Gel properties were visually evaluated and rated as follows, with the results shown in Table I:
- Short Flow ("SF") indicates a mayonnaise-like consistency in which unconfined formulations on a flat surface maintain their static state structural integrity.
- Long Flow ("LF") indicates a honey-like consistency in which unconfined formulations on a flat surface flow and do not maintain their static state structural integrity.
- Runny Flow ("R") indicates water like consistency.

**TABLE I**

| Example number | Heat treatment time (minutes) | Viscosity (mPas) | Rheology |
|---|---|---|---|
| 10-1 | 0 | 1300 | long flow |
| 10-2 | 35 | 16,200 | Short flow |
| 10-3 | 40 | 18,400 | Short flow |
| 10-4 | 45 | 17,900 | Short flow |
| 10-5 | 50 | 17,500 | Short flow |
| 10-6 | 60 | 15,800 | Short flow |
| 10-7 | 75 | 7,800 | Short flow |
| 10-8 | 0 | <100 | Runny flow |

These data indicate that for formulations in which the polymer is neutralized the heat treatment gives the short flow characteristics desirable for a gel formulation. It also gives a large increase in viscosity. There is no increase in viscosity (Runny flow) if the neutralizing agent is not added and the pH of the hair gel is not raised to 6.5-7.5 range (Example 10-8).

### Example 11: Evaluation of Rheology

The spray-dried rheology modifier powder of Example 7 was heat treated in a 130°C oven for 60 minutes. A hair gel formulation was made as in Example 9 using 2 weight percent of this heat treated material and triethanolamine as the neutralizing agent to adjust the pH of the formulation to 7. The resulting hair gel was clear and had a viscosity of 58,740 (mPas). It also had the short flow rheology characteristic desirable for a gel product.

### Example 12: Polysaccharide present during polymerization reaction; Evaluation of Rheology

1291.6 grams of water was charged into a reactor and heated to 82°C. An overhead nitrogen sparge was started upon initial heating of the water. When the water was fully heated, 92.3 g of 88.4% PR1303A (weight average molecular weight of 2,259,000, from Avebe) potato starch was added to the reactor. The starch was cooked for 30 minutes at 82°C. A monomer solution containing 104.3 grams ethyl acrylate, 134.8 grams methacrylic acid, 20 grams C₁₆₋₁₈ alcohol with 20 moles ethoxylate methacrylate (associative monomer) and 0.976 grams trimethylolpropane triacrylate was subsequently added to the reactor over a period of 90 minutes. An initiator solution comprised of 1.02 grams ammonium persulfate and 146.8 grams water was added to the reactor at the same time as the monomer solutions over a period of 90 minutes. The reaction product was held at 82°C for an additional 120 minutes. The heat was turned off and nitrogen was blown over the sample during cooling to remove ethyl acrylate. The final product was a homogeneous, white emulsion with an average particle size of 670 nm which was then spray dried to form a non-tacky white powder. Samples of this powder were then heat treated for different times and at either 110°C or 120°C. Hair gels were then made with these materials using the procedure detailed in Example 9 and using 1% of the dried rheology modifier. The viscosity of these hair gels were measured and are reported below in Table II.

**TABLE II**

| Time of heat treatment (minutes) | Viscosity (mPas), samples heat treated at 110°C | Rheology | Viscosity (mPas), samples heat treated at 120°C | Rheology |
|---|---|---|---|---|
| 0 | 10,500 | Long flow | 11,200 | Long flow |
| 15 | 15,500 | Short flow | 16,600 | Short flow |
| 30 | 17,200 | Short flow | 16,800 | Short flow |
| 45 | 16,200 | Short flow | 15,900 | Short flow |
| 60 | 15,400 | Short flow | 16,900 | Short flow |
| 90 | 17,500 | Short flow | 18,300 | Short flow |

### Example 13: Polymer blended with polysaccharide

452 grams of water and 4.65 g of SYNPERONIC® A-50 (Croda) surfactant were charged into a 1 liter reactor fitted with an agitator, condenser, thermometer and heated to 70°C. An overhead nitrogen sparge was started upon initial heating. When the temperature was reached, 79 g of ethyl acrylate, 52.6 g of N'N-dimethyl aminoethylmethacrylate, 6.9 g of cetyl 20 EO itaconate associative monomer, 0.069 g of diallylphthalate and 8.2 g of isopropanol were premixed and added to the reactor with mixing to form an emulsion. A first initiator solution containing 0.83 g of sodium persulfate dissolved in 35.4 g of deionized water was prepared. A second initiator solution containing 2.4 g of sodium bisulfite (41% aqueous solution) dissolved in 50 g of deionized water was also prepared. Both of these initiator solutions were slowly added to the reactor over 2 hours while the reactor contents were maintained at 70°C. A scavenge initiator solution containing 0.51 g of sodium persulfate dissolved in 9 g of water was added over 30 minutes. The reactor contents were held at 70°C for 1 hour. The reactor contents were cooled to 25°C. Next 35 g of DE 10 maltodextrin (StarDri® 100 from Tate and Lyle) was added to the reaction mixture and stirred for 2 hours. This solution was then spray dried to form a non-tacky white powder. A sample of the emulsion polymerization reaction product taken prior to the addition of the maltodextrin formed a sticky mess and could not be spray dried.

### Example 14: Polymer blended with polysaccharide

452 grams of water and 4.65 g of SYNPERONIC® A-50 surfactant were charged into a 1 liter reactor fitted with an agitator, condenser, and thermometer, and heated to 70°C. An overhead nitrogen sparge was started upon initial heating. When the temperature was reached, 79 g of ethyl acrylate, 52.6 g of N'N-dimethyl aminoethylmethacrylate, 6.9 g of cetyl 20 EO itaconate associative monomer, 0.069 g of diallylphthalate and 8.2 g of isopropanol were premixed and added to the reactor with mixing to form an emulsion. A first initiator solution containing 0.83 g of sodium persulfate dissolved in 35.4 g of deionized water was prepared. A second initiator solution containing 2.4 g of sodium bisulfite (41% aqueous solution) dissolved in 50 g of deionized water was also prepared. Both of these initiator solutions were slowly added to the reactor over 2 hours while the reactor contents were maintained at 70°C. A scavenge initiator solution containing 0.51 g of sodium persulfate dissolved in 9 g of water was added over 30 minutes. The reactor contents were held at 70°C for 1 hour. The reactor contents were cooled to 25°C. Next 70 g of DE 10 maltodextrin was added to the reaction mixture and stirred for 2 hours. This solution was then spray dried to form a non-tacky white powder. Samples of the powder were heat treated at 130°C for 30 and 45 minutes, respectively. The resulting heat treated product (2-5 weight%) was dispersed in water and neutralized with acetic acid to a pH of 4. The resulting gels had a short flow character.

### Example 15: Polymer blended with polysaccharide

452 grams of water and 4.65 g of SYNPERONIC® A-50 surfactant were charged into a 1 liter reactor fitted with an agitator, condenser, thermometer and heated to 70°C. An overhead nitrogen sparge was started upon initial heating. When the temperature was reached, 79 g of ethyl acrylate, 52.6 g of N'N-dimethyl aminoethylmethacrylate, 6.9 g of cetyl 20 EO itaconate associative monomer, 0.069 g of diallylphthalate and 8.2 g of isopropanol were premixed and added to the reactor with mixing to form an emulsion. A first initiator solution containing 0.83 g of sodium persulfate dissolved in 35.4 g of deionized water was prepared. A second initiator solution containing 2.4 g of sodium bisulfite (41% aqueous solution) dissolved in 50 g of deionized water was also prepared. Both of these initiator solutions were slowly added to the reactor over 2 hours while the reactor contents were maintained at 70°C. An initiator solution was used as a polishing step to polymerize remaining monomer so as to reduced residual monomer levels to below 1000 ppm; the solution contained 0.51 g of sodium persulfate dissolved in 9 g of water and was added over 30 minutes. The reactor contents were held at 70°C for 1 hour. The reactor contents were cooled to 25°C. Next 540.05 g of DE 10 maltodextrin was added to the reaction mixture and stirred for 2 hours. Then 13.85 g of Aerosil® 380 was added to the reaction mixture. This solution was then spray dried to form a non-tacky white powder.

### Example 16: Polysaccharide present during polymerization reaction

641 grams of water and 64.3 g of 80.9% Perfectamyl® 4692 potato starch with a Mw of 689,000 (from Avebe) was charged into a reactor and heated to 82°C. An overhead nitrogen sparge was started upon initial heating of the water. The starch was cooked for 30 minutes at 82°C. A monomer solution containing 25.1 g of acrylic acid, 7.8 g of hydroxypropyl methacrylate, 54.9 g of methyl methacrylate and 62.7 g of tertiary octyl acrylamide was subsequently added to the reactor over a period of 90 minutes. An initiator solution containing 0.656 gram of ammonium persulfate and 65 grams water was added to the reactor at the same time as the monomer solutions over a period of 90 minutes. The reaction product was held at 82°C for an additional 120 minutes. The final product was a homogeneous, white emulsion and was then spray dried to produce a non-tacky white powder.

### Example 17: Polyvinyl alcohol present during polymerization reaction

600 grams of water and 20.5 g of polyvinyl alcohol Kuraray Poval® 5-88 was charged into a reactor and heated to 82°C. An overhead nitrogen sparge was started upon initial heating of the water. A monomer solution containing 25.1 g of acrylic acid, 7.8 g of hydroxypropyl methacrylate, 54.9 g of methyl methacrylate and 62.7 g of tertiary octyl acrylamide was subsequently added to the reactor over a period of 90 minutes. An initiator solution containing 0.656 gram of ammonium persulfate and 65 grams water was added to the reactor at the same time as the monomer solutions over a period of 90 minutes. The reaction product was held at 82°C for an additional 120 minutes. The final product was a homogeneous, white emulsion and was then spray dried to produce a non-tacky white powder.

### Example 18: Blend of alkali swellable with starch

997.4 grams of water and 16.3 of Stanfax 234 (30% aqueous solution of sodium lauryl sulfate) was charged into a reactor and heated to 82°C. An overhead nitrogen sparge was started upon initial heating of the water. A monomer solution containing 336.6 grams ethyl acrylate and 173.4 grams methacrylic acid was subsequently added to the reactor over a period of 90 minutes. An initiator solution containing 1.6 gram of ammonium persulfate and 121.8 grams water was added to the reactor at the same time as the monomer solutions over a period of 90 minutes. The reaction product was held at 82°C for an additional 120 minutes. The reaction product was heated to 85°C. A solution of 170 grams of DE 10 maltodextrin dissolved in 1540 g of water was slowly added at 85°C. This mixture was stirred at 85°C for 2 hours. The final product was a homogeneous, white emulsion with a pH of 2.8. This was then spray dried to produce a non-tacky white powder.

### Example 19: Blend of alkali swellable with starch

997.4 grams of water and 16.3 of Stanfax 234 (30% aqueous solution of sodium lauryl sulfate) was charged into a reactor and heated to 82°C. An overhead nitrogen sparge was started upon initial heating of the water. A monomer solution containing 336.6 grams ethyl acrylate and 173.4 grams methacrylic acid was subsequently added to the reactor over a period of 90 minutes. An initiator solution containing 1.6 gram of ammonium persulfate and 121.8 grams water was added to the reactor at the same time as the monomer solutions over a period of 90 minutes. The reaction product was held at 82°C for an additional 120 minutes. The reaction product was heated to 85°C. A solution of 170 grams of DE 10 maltodextrin dissolved in 1540 g of water was slowly added at 85°C. This mixture was stirred at 85° C for 2 hours. The pH was adjusted to 3.5 by adding 6 g of 5% sodium hydroxide solution. This was then spray dried to produce a non-tacky white powder.

### Example 20: Blend of alkali swellable with starch

997.4 grams of water and 16.3 of Stanfax 234 (30% aqueous solution of sodium lauryl sulfate) was charged into a reactor and heated to 82°C. An overhead nitrogen sparge was started upon initial heating of the water. A monomer solution containing 336.6 grams ethyl acrylate and 173.4 grams methacrylic acid was subsequently added to the reactor over a period of 90 minutes. An initiator solution containing 1.6 gram of ammonium persulfate and 121.8 grams water was added to the reactor at the same time as the monomer solutions over a period of 90 minutes. The reaction product was held at 82°C for an additional 120 minutes. The reaction product was heated to 85°C. A solution of 170 grams of DE 10 maltodextrin dissolved in 1540 g of water was slowly added at 85°C. This mixture was stirred at 85°C for 2 hours. The pH was adjusted to 5 by adding approximately 15 g of 5% sodium hydroxide solution. This was then spray dried to produce a non-tacky white powder.

### Example 21: Blend with another swellable emulsion polymer

100 grams of the spray-dried rheology modifier of Example 1 is blended with 100 grams of Alcogum® L11, a surfactant stabilized alkali swellable emulsion polymer from AkzoNobel Surface Chemistry. The blended product is spray dried to produce a non-tacky white powder.

### Example 22: Acid swellable reacted with polysaccharide

688 grams of water and 29 grams of 82% Perfectamyl® A4692 potato starch (from Avebe) was added to a reactor fitted with an agitator, condenser, and thermometer, and heated to 82°C. Nitrogen was bubbled subsurface upon initial heating. The granular starch was cooked at 82°C for 60 minutes to make the starch water soluble. The starch solution was cooled to 43C, and the pH of the starch solution was raised from approximately 7 to pH 9 using 1.4 grams 99% triethanolamine. Next, 82.6 g of ethyl acrylate, 51.6 g of N'N-dimethyl aminoethylmethacrylate, 0.65 g of triethanolamine, and 7 g of cetyl 20 EO itaconate associative monomer were mixed and added to the reactor over 90 minutes. Two initiator solutions, one containing 0.37 g of sodium persulfate dissolved in 40 g of water, and one containing 1.1 g of sodium bisulfite (41%) dissolved in 35 g of water, were simultaneously added to the reactor over 90 minutes. The resulting emulsion was held at 43°C for 1 hour. This emulsion was then spray dried to form a non-tacky white powder. A sample containing 10% active emulsion was made, and the pH was lowered to 3.5 with glycolic acid. This sample gave a viscosity of 984 mPas.

### Example 23: Alkali swellable reacted with polysaccharide

706.9 grams of water was charged into a reactor and heated to 82°C. An overhead nitrogen sparge was started upon initial heating of the water. 84.4 g of 80.9% PR1004B potato starch with a Mw of 862,000 Daltons (from Avebe) was charged into the reactor. The starch was cooked for 30 minutes at 82°C. A monomer solution containing 42.6 g of ethyl acrylate and 24.6 g of methacrylic acid was subsequently added to the reactor over a period of 30 minutes. An initiator solution containing 0.29 grams of ammonium persulfate and 37.6 grams water was added to the reactor at the same time as the monomer solutions over a period of 30 minutes. The reaction product was held at 82°C for an additional 60 minutes. The final product was a homogeneous, white emulsion with a solids content of 14.46% and a Mw of 1551000 Daltons. This emulsion was then spray dried to form a non-tacky white powder.

### Example 24: Alkali swellable reacted with polysaccharide

710.4 grams of water was charged into a reactor and heated to 82°C. An overhead nitrogen sparge was started upon initial heating of the water. 42 g of 80.9% PR1004B potato starch with a Mw of 862,000 Daltons (from Avebe) was charged into the reactor. The starch was cooked for 30 minutes at 82°C. A monomer solution containing 63.7 g of ethyl acrylate and 36.9 g of methacrylic acid was subsequently added to the reactor over a period of 90 minutes. An initiator solution containing 0.42 grams of ammonium persulfate and 42.2 grams water was added to the reactor at the same time as the monomer solutions over a period of 90 minutes. The reaction product was held at 82°C for an additional 60 minutes. The final product was a homogeneous, white emulsion with a solids content of 13.84 and a Mw of 1649000 Daltons. This emulsion was then spray dried to form a non-tacky white powder.

### Example 25: Alkali swellable reacted with polysaccharide

706.9 grams of water was charged into a reactor and heated to 82°C. An overhead nitrogen sparge was started upon initial heating of the water. 84.4 g of 80.9% PR1004B potato starch with a Mw of 862,000 Daltons (from Avebe) was charged into the reactor. The starch was cooked for 30 minutes at 82°C. A monomer solution containing 42.6 g of ethyl acrylate and 24.6 g of methacrylic acid was subsequently added to the reactor over a period of 90 minutes. An initiator solution containing 0.29 grams of ammonium persulfate and 37.6 grams water was added to the reactor at the same time as the monomer solutions over a period of 90 minutes. The reaction product was held at 82°C for an additional 60 minutes.

The final product was a homogeneous, white emulsion with a solids content of 13.79 and a Mw of 2266000 Daltons. This emulsion was then spray dried to form a non-tacky white powder.

### Example 26: Acid swellable reacted with polysaccharide

45.1 grams of DE 10 maltodextrin were dissolved in 603 grams of water in a 1 liter reactor fitted with an agitator, condenser, thermometer and heated to 65°C and sparged with subsurface N2 for 60 minutes. 10 grams of triethanolamine was then added. 7.7 grams of Polystep® TD 507 (Stepan Company) surfactant was added next. Next 7.8 g of cetyl 20 EO itaconate associative monomer was added. The temperature was then lowered to 55C and 91.6 g of ethyl acrylate was added over 10 minutes. Next 55.8 g of N'N-dimethyl aminoethylmethacrylate was mixed with 8.4 grams of propylene glycol and added to the reactor in 10 minutes. A first initiator solution containing 0.96 g of sodium persulfate dissolved in 30 g of deionized water was added over 90 minutes. A second initiator solution containing 2.8 g of sodium bisulfite (41% aqueous solution) dissolved in 27 g of deionized water was added over 90 minutes. The reaction was not allowed to exotherm over 60C and was cooled if necessary. At the end of these feeds an initiator solution containing 0.6 g of sodium persulfate dissolved in 17 g of water was added over 60 minutes while holding the temperature at 60C. The reactor contents were held at 70°C for 1 hour. This emulsion polymer was spray dried to non-tacky white powder. The final dried product contained 75% active emulsion polymer.

### Example 27 Acid swellable reacted with polysaccharide

67.7 grams of DE 18 maltodextrin were dissolved in 603 grams of water in a 1 liter reactor fitted with an agitator, condenser, thermometer and heated to 65°C and sparged with subsurface N2 for 60 minutes. 7.7 grams of Polystep® TD 507 surfactant was added next. Next 7.8 g of cetyl 20 EO itaconate associative monomer was added. 1.7 grams of triethanolamine and 0.6 grams of 50% NaOH was then added. The temperature was then lowered to 55C and 91.6 g of ethyl acrylate was added over 10 minutes. Then 8.4 grams of propylene glycol was added. Next 55.8 g of N'N-dimethyl aminoethylmethacrylate was and added to the reactor in 10 minutes. A first initiator solution containing 0.96 g of sodium persulfate dissolved in 30 g of deionized water was added over 90 minutes. A second initiator solution containing 2.8 g of sodium bisulfite (41% aqueous solution) dissolved in 27 g of deionized water was added over 90 minutes. The reaction was not allowed to exotherm over 60C and was cooled if necessary. At the end of these feeds an initiator solution containing 0.6 g of sodium persulfate dissolved in 17 g of water was added over 60 minutes while holding the temperature at 60C. The reactor contents were held at 70°C for 1 hour. This emulsion polymer was spray dried to a non-tacky white powder. The final dried product contained 66% active emulsion polymer.

### Example 28: Acid swellable reacted with polysaccharide

41.2 grams of DE 10 maltodextrin were dissolved in 630 grams of water in a 1 liter reactor fitted with an agitator, condenser, thermometer and heated to 65°C and sparged with subsurface N2 for 60 minutes. 5.7 grams of Polystep® TD 507 surfactant was added next. Next 4.8 g of cetyl 20 EO itaconate associative monomer was added. 1.0 grams of triethanolamine and 0.4 grams of 50% NaOH was then added. The temperature was then lowered to 55C and 55.7 g of ethyl acrylate was added over 10 minutes. Then 5.1 grams of propylene glycol was added. Next 33.9 g of N'N-dimethyl aminoethylmethacrylate was and added to the reactor in 10 minutes. A first initiator solution containing 0.59 g of sodium persulfate dissolved in 44 g of deionized water was added over 90 minutes. A second initiator solution containing 1.7 g of sodium bisulfite (41% aqueous solution) dissolved in 43.2 g of deionized water was added over 90 minutes. The reaction was not allowed to exotherm over 60C and was cooled if necessary. At the end of these feeds an initiator solution containing 0.36 g of sodium persulfate dissolved in 17 g of water was added over 60 minutes while holding the temperature at 60C. The reactor contents were held at 70°C for 1 hour. This emulsion polymer was spray dried to non-tacky white powder.

### Example 29 Acid swellable reacted with polysaccharide

60.52 grams of 80.9% Perfectamyl® 4692 potato starch with a Mw of 689,000 (from Avebe) were dispersed in 715 grams of water in a 1 liter reactor fitted with an agitator, condenser, thermometer and heated to 82°C and sparged with subsurface N2 for 60 minutes. The reactor was then cooled to 71C. 5.7 grams of Polystep® TD 507 surfactant was added next. Next 5.8 g of cetyl 20 EO itaconate associative monomer was added. 1.2 grams of triethanolamine and 0.45 grams of 50% NaOH was then added. The temperature was then lowered to 55C and 68 g of ethyl acrylate was added over 10 minutes. Then 6.3 grams of propylene glycol was added. Next 41.5 g of N'N-dimethyl aminoethylmethacrylate was and added to the reactor in 10 minutes. A first initiator solution containing 0.72 g of sodium persulfate dissolved in 22 g of deionized water was added over 90 minutes. A second initiator solution containing 2.1 g of sodium bisulfite (41% aqueous solution) dissolved in 43.2 g of deionized water was added over 90 minutes. The reaction was not allowed to exotherm over 60C and was cooled if necessary. At the end of these feeds an initiator solution containing 0.44 g of sodium persulfate dissolved in 17 g of water was added over 60 minutes while holding the temperature at 60C. The reactor contents were held at 70°C for 1 hour. This emulsion polymer was spray dried to non-tacky white powder.

### Example 30 Acid swellable reacted with polysaccharide

45.2 grams of DE 10 maltodextrin (90.4%) were dissolved in 603 grams of water in a 1 liter reactor fitted with an agitator, condenser, thermometer and heated to 65°C and sparged with subsurface N2 for 60 minutes. 7.7 grams of Polystep® TD 507 surfactant was added next. Next 7.8 g of cetyl 20 EO itaconate associative monomer was added. 1.7 grams of triethanolamine was then added. The temperature was then lowered to 55C and 91.6 g of ethyl acrylate was added over 10 minutes. Then 8.4 grams of propylene glycol was added. Next 55.8 g of N'N-dimethyl aminoethylmethacrylate was and added to the reactor in 10 minutes. A first initiator solution containing 0.96 g of sodium persulfate dissolved in 30 g of deionized water was added over 90 minutes. A second initiator solution containing 2.8 g of sodium bisulfite (41% aqueous solution) dissolved in 27 g of deionized water was added over 90 minutes. The reaction was not allowed to exotherm over 60C and was cooled if necessary. At the end of these feeds an initiator solution containing 0.6 g of sodium persulfate dissolved in 17 g of water was added over 60 minutes while holding the temperature at 60C. The reactor contents were held at 70°C for 1 hour. This emulsion polymer was spray dried to a white powder. The final dried product contained 80% active emulsion polymer.

### Example 31 (comparative example):

603 grams of water was placed in a 1 liter reactor fitted with an agitator, condenser, thermometer and heated to 65°C and sparged with subsurface N2 for 60 minutes. 7.7 grams of Polystep® TD 507 surfactant was added next. Next 7.8 g of cetyl 20 EO itaconate associative monomer was added. 1.7 grams of triethanolamine was then added. The temperature was then lowered to 55C and 91.6 g of ethyl acrylate was added over 10 minutes. Then 8.4 grams of propylene glycol was added. Next 55.8 g of N'Ndimethyl aminoethylmethacrylate was and added to the reactor in 10 minutes. A first initiator solution containing 0.96 g of sodium persulfate dissolved in 30 g of deionized water was added over 90 minutes. A second initiator solution containing 2.8 g of sodium bisulfite (41% aqueous solution) dissolved in 27 g of deionized water was added over 90 minutes. The reaction was not allowed to exotherm over 60C and was cooled if necessary. At the end of these feeds an initiator solution containing 0.6 g of sodium persulfate dissolved in 17 g of water was added over 60 minutes while holding the temperature at 60C. The reactor contents were held at 70°C for 1 hour. Upon cooling attempts were made to dry this polymer, however it was not possible to form a non-tacky powder.

### Example 32:

Samples of the synthetic polymers of Examples 30 and 31 were each dispersed in water at 1% active and then neutralized with 70% glycolic acid to a pH or approximately 3.5 and the viscosity was measured, as shown in Table III.

**TABLE III**

| Polymer | Viscosity of neutralized 1% active synthetic polymer (mPas) | pH |
|---|---|---|
| Example 30 liquid | 4340 | 3.7 |
| Example 30 spray dried | 3900 | 3.4 |
| Example 31 | 1270 | 3.5 |

These viscosity data indicate surprisingly that even in the liquid form Example 30 is more efficient than Comparative Example 31 made without maltodextrin in the initial charge. Both the liquid and solid versions of Example 30 have better viscosity performance than Comparative Example 31.

### Example 33 Acid swellable reacted with polyvinyl alcohol

44.7 grams of polyvinyl alcohol (Poval 5-88 Kuraray) was added to 603 grams of water in a 1 liter reactor fitted with an agitator, condenser, thermometer and heated to 65°C and sparged with subsurface N2 for 60 minutes. 7.7 grams of Polystep® TD 507 surfactant was added next. Next 7.8 g of cetyl 20 EO itaconate associative monomer was added. 1.7 grams of triethanolamine and 0.6 grams of 50% NaOH was then added. The temperature was then lowered to 55C and 91.6 g of ethyl acrylate was added over 10 minutes. Then 8.4 grams of propylene glycol was added. Next 55.8 g of N'N-dimethyl aminoethylmethacrylate was and added to the reactor in 10 minutes. A first initiator solution containing 0.96 g of sodium persulfate dissolved in 30 g of deionized water was added over 90 minutes. A second initiator solution containing 2.8 g of sodium bisulfite (41% aqueous solution) dissolved in 27 g of deionized water was added over 90 minutes. The reaction was not allowed to exotherm over 60C and was cooled if necessary. At the end of these feeds an initiator solution containing 0.6 g of sodium persulfate dissolved in 17 g of water was added over 60 minutes while holding the temperature at 60C. The reactor contents were held at 70°C for 1 hour. This emulsion polymer was spray dried to non-tacky white powder. The final dried product contained 77% active emulsion polymer.

### Example 34: Polysaccharide present during polymerization reaction

1185 grams of water and 84.7 g of 88.4% PR1303A potato starch (weight average molecular weight of 2,259,000) (from Avebe) were charged into a reactor and heated to 82°C. An overhead nitrogen sparge was started upon initial heating of the water. The starch solution was cooked for 30 minutes at 82°C. A monomer solution containing 70.7 grams ethyl acrylate, 25.0 grams of methyl methacrylate, 122.3 grams methacrylic acid, and 0.895 grams trimethylolpropane triacrylate was subsequently added to the reactor over a period of 90 minutes. An initiator solution containing 0.94 grams ammonium persulfate and 134.7 grams water was added to the reactor at the same time as the monomer solution over a period of 90 minutes. The reaction product was held at 82°C for an additional 120 minutes. The heat was turned off and nitrogen was blown over the sample during cooling to remove ethyl acrylate, during which time an additional 39 grams of water was added to the reactor. The final product was a homogeneous, white emulsion with a total solids content of around 17.5 weight% and a pH of 2.4 and an average particle size of 380 nm. This reaction product was then spray dried to produce a non-tacky white powder.

### Example 35: Acid swellable reacted with polysaccharide

120.7 grams of DE 10 waxy maltodextrin (95 %) were dissolved in 415 grams of water in a 2 liter reactor fitted with an agitator, condenser, thermometer and heated to 60 °C and sparged with subsurface N2 for 60 minutes. 5.3 grams of Polystep® TD 507 surfactant was added next. Next 5.4 g of cetyl 20 EO itaconate associative monomer was added. 1.2 grams of triethanolamine was then added. The temperature was then lowered to 55C and 63.1 g of ethyl acrylate was added over 10 minutes. Then 5.8 grams of propylene glycol was added. Next 38.5 g of N'N-dimethyl aminoethylmethacrylate was added to the reactor in 10 minutes. A first initiator solution containing 0.66 g of sodium persulfate dissolved in 250 g of deionized water was added over 90 minutes. A second initiator solution containing 1.94 g of sodium bisulfite (41% aqueous solution) dissolved in 258 g of deionized water was added over 90 minutes. The reaction was not allowed to exotherm over 60C and was cooled if necessary. At the end of these feeds an initiator solution containing 0.4 g of sodium persulfate dissolved in 12 g of water was added over 60 minutes while holding the temperature at 60C. The reactor contents were held at 70°C for 1 hour. This emulsion polymer was spray dried to a non-tacky white powder and the polymer did not stick to the sides of the spray dryer at all. The final dried product contained 50% active emulsion polymer.

### Example 36: Polysaccharide present during polymerization reaction

1348.6 grams of water was charged into a reactor and heated to 82C. An overhead nitrogen sparge was started upon initial heating of the water. 100.1 g of 80.9 % 1004B potato starch (from Avebe) was added to the reactor when temperature had reached 82C. The starch was cooked for 30 minutes at 82°C. A monomer solution containing 151.6 grams ethyl acrylate, 87.7 grams methacrylic acid, and 0.954 grams trimethylolpropane triacrylate was subsequently added to the reactor over a period of 90 minutes. At the end of the 90 minutes, 5 grams of rinse water was added to the reactor through the same feed tube as fed the monomer solution. An initiator solution containing 1 gram of ammonium persulfate and 100.5 grams water was added to the reactor at the same time as the monomer solution over a period of 90 minutes. The reaction product was held at 82°C for an additional 120 minutes. The reactor contents were allowed to cool to ambient temperature and the rotation speed of the overhead stirrer was increased from 145 rpm to 260 rpm. 14.25 g of 10% sodium hydroxide solution was added over a period of 20 minutes. The sample continued to mix for 30 minutes, then 89.91 g of 10% sodium bisulfite solution was added over 60 minutes. 2.8 grams of Proxel GXL was added to the reactor contents. The final product was a homogeneous, white emulsion with a total solids content of around 16.6 % and a pH of 5.0. This reaction product was then spray dried to produce a non-tacky white powder with an average particle size of around 10-20 microns.

### Example 37: Effect of duration of heat treatment on product viscosity in hair gel

Samples of the spray dried rheology modifier of Example 36 were subjected to heat treatment as follows: A disposable aluminum pan was obtained measuring approximately 3 inches in width by 5 inches in length by 2 inches in height. 10 grams of the spray dried powdered sample of Example 36 was placed evenly into this aluminum pan, which was placed in a 130°C oven at various times and then removed and placed into typical screw top glass jar. Samples were heated in this manner for durations of 15, 30, 45, 60 and 90 minutes.

### Preparation of hair gel samples

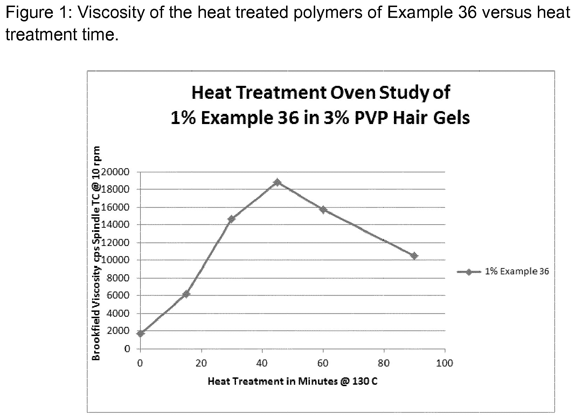

Each of the heat treated samples prepared as described above, and a control sample of the same spray dried rheology modifier that had not been heat treated, were used to prepare hair gel formulations in accordance with Example 9 using the following procedure:
A glass beaker was obtained and 54.33 grams of water was added. The beaker was placed under an overhead mixer using a 1.5 inch Jiffy mixer blade. 1.0 gram of the above heat treated sample or control sample was then taken and slowly added to the 54.33 grams of water with a vortex extending to the bottom of the beaker. The sample was allowed to mix until it was uniform and homogenous with no visible lumps. In a separate beaker 41 grams of water was added with a stir bar, and mixed with a vortex extending to the bottom of the beaker. Then 3.0 grams polyvinylpyrridone K90 (Ashland Specialty Products, Bridgewater, NJ) was slowly added and mixed until uniform and clear. Next -0.17 grams of 99% Triethanolamine (Fisher Chemical, Fair Lawn, NJ) was added and mixed until uniform. The contents of the beaker containing water, polyvinylpyrridone K90, and Triethanolamine was combined with the main beaker containing the 1.0 gram of heat treated polymer and water. This was allowed to mix until uniform and then 0.5 grams of Glydant Plus® (DMDM Hydantoin and lodopropynyl Butylcarbamate) 1,3-Dihydroxylmethyl-5, 5-Dimethylhydantoin and 3-lodo-2-Propynyl Butyl Carbamate (LONZA Corporation, Allendale, NJ) was added and mixed until uniform. More Triethanolamine was added until the pH reached ∼ 6.8. The samples were measured for viscosity using a Brookflield RVDV-1 Prime using spindle TC at 10 rpm with the helipath at 25°. The viscosity results are shown in Table IV, and illustrated in Figure 1.

**Table IV: 3% Polyvinylpyrridone K90 Hair Gels made with 1% Polymers of Example 36**

| **Sample ID** | **Heat Treatment (Minutes @130°C)** | **pH** | **Viscosity cps TC/(10rpm)** |
|---|---|---|---|
| 37-1 | 0 | 6.89 | 1700 |
| 37-2 | 15 | 6.83 | 6200 |
| 37-3 | 30 | 6.92 | 14700 |
| 37-4 | 45 | 6.85 | 18800 |
| 37-5 | 60 | 6.85 | 15700 |
| 37-6 | 90 | 6.97 | 10500 |

The data in Table IV and Figure 1 illustrate that heat treatment has an effect on the viscosity of the formulation. Without heat treatment the rheology modifier achieved 1,700 cps using a Brookfield RVDV-1 Prime viscometer spindle TC @10 rpm at 25°C. The rheology modifier that was heat treated for 45 minutes at 130°C showed that the viscosity increased to 18,800 cps Brookfield Viscosity RVDV-1 Prime viscometer spindle TC @ 10 rpm at 25°C.

### Example 38: Polysaccharide present during polymerization reaction

458.5 grams of water was charged into a reactor and heated to 82C. An overhead nitrogen sparge was started upon initial heating of the water. 31.1 g of 88.4 % 1303A potato starch (from Avebe) was added to the reactor when temperature had reached 82C. The starch was cooked for 60 minutes at 82°C. A monomer solution containing 41.2 grams ethyl acrylate, 22.2 grams methacrylic acid, 10.3 grams methyl methacrylate, 18.3 grams of a 36% solution of branched C20 alcohol 22 EO methacrylate in water, and 1.2886 grams trimethylolpropane triacrylate was subsequently added to the reactor over a period of 90 minutes. At the end of the 90 minutes, 5 grams of rinse water was added to the reactor through the same feed tube as fed the monomer solution. An initiator solution containing 0.61 gram of ammonium persulfate and 35 grams water was added to the reactor at the same time as the monomer solution over a period of 20 minutes. After the completion of the initiator feed, 540 grams water was added subsurface over 70 minutes, the end of which coincided with the end of the monomer solution addition. The reaction product was held at 82°C for an additional 60 minutes. 1 gram of Proxel GXL was added to the reactor contents. The final product was a homogeneous, white emulsion with a total solids content of around 9.76. This reaction product was then spray dried to produce a non-tacky white powder with an average particle size of around 10-20 microns.

### Example 39: Polysaccharide present during polymerization reaction

680 grams of water and 57.9 g of 80.9 % PR1004B potato starch (from Avebe) were charged into a reactor. An additional 100 grams of water was added to the reactor and the contents were heated to 82°C. An overhead nitrogen sparge was started upon heating the water. The starch was cooked for 60 minutes at 82°C. An agitated monomer solution containing 87.6 grams ethyl acrylate, 50.7 grams methacrylic acid, and 0.5518 grams trimethylolpropane triacrylate was subsequently added to the reactor over a period of 90 minutes. An initiator solution containing 0.58 gram of ammonium persulfate and 58.1 grams water was added to the reactor at the same time as the monomer solution over a period of 90 minutes. The reaction product was held at 82°C for an additional 120 minutes. The reactor contents were allowed to cool to ambient temperature. 8.25 g of 10% sodium hydroxide solution was added over a period of 20 minutes. The sample continued to mix for 30 minutes, then 52 grams of 10% sodium bisulfite solution was added over 60 minutes. 1 gram of Proxel GXL was added to the reactor contents. The final product was a homogeneous, white emulsion with a total solids content of around 15.5%. This reaction product was then spray dried to produce a non-tacky white powder with an average particle size of around 10-20 microns.

### Example 40: Effect of duration of oven heat treatment on solution viscosity

Samples of the rheology modifiers of Examples 38 and 39 were heat treated at 130°C in accordance with the procedure of Example 37 for periods of 30 minutes, 60 minutes and 120 minutes. A control sample of each rheology modifier was not heat treated.

Test formulations using each of the test samples were prepared substantially in accordance with the method of Example 37, but without the polyvinylpyrrolidone. These samples were made by placing 95.83 grams of water into a breaker and mixing it using a 1.5 inch Jiffy mixer blade. This was mixed with a vortex extending to the bottom of the beaker and 3.5 grams of the rheology modifier was slowly added. This was mixed until uniform and then 0.17 grams of 99% Triethanolamine (Fisher Chemical, Fair Lawn, NJ) was added. Mixing was continued until the mixture was homogenous and uniform. To this mixture was added 0.5 grams of Glydant Plus® (DMDM Hydantoin and lodopropynyl Butylcarbamate) 1,3-Dihydroxylmethyl-5, 5-Dimethylhydantoin and 3-lodo-2-Propynyl Butyl Carbamate (LONZA Corporation, Allendale, NJ) was added as a preservative agent. The solution was then transferred to a screw top glass jar and centrifuged and then measured for viscosity using a Brookfield RVDV-1 PRIME spindle TC @ 10rpm at 25°C. The viscosity results are presented in Table V.

**Table V: Effect of Oven Heat Treatment on Samples without polyvinylpyrridone K90**

| **Sample** | **Heat Treatment (Minutes @130°C)** | **pH** | **Viscosity Spindle cps TC / (10 rpm)** |
|---|---|---|---|
| 3.5% Polymer of Example 38 | 0 | 7.51 | 33000 |
| 3.5% Polymer of Example 38 | 30 | 6.57 | 54000 |
| 3.5% Polymer of Example 38 | 60 | 6.54 | 54900 |
| 3.5% Polymer of Example 38 | 120 | 6.86 | 64200 |
| 3.5% Polymer of Example 39 | 0 | 8.01 | 9500 |
| 3.5% Polymer of Example 39 | 30 | 6.95 | 88250 |
| 3.5% Polymer of Example 39 | 60 | 6.74 | 69000 |
| 3.5% Polymer of Example 39 | 120 | 6.89 | 241000* |

| | | | |
|---|---|---|---|
| * This viscosity was taken using spindle TC / (5 rpm) due to the extremely high viscosity of this sample. | | | |

For each rheology modifier the viscosity of the formulation increased with the duration of the heat treatment of the rheology modifier.

### Example 41: Effect of heat treatment at different temperatures

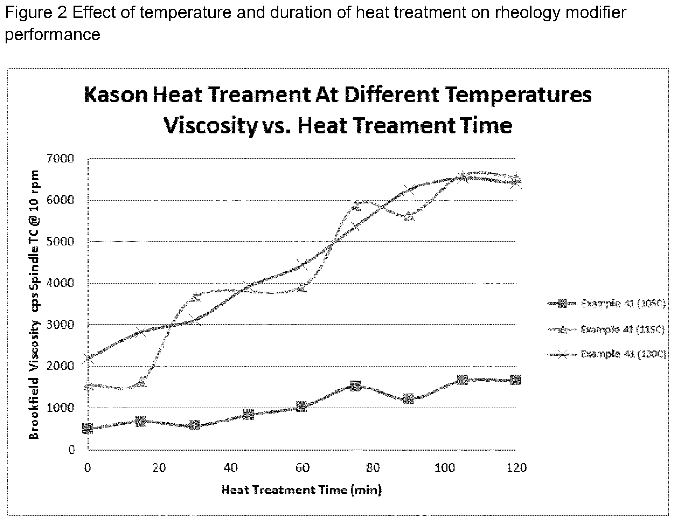

A polymer made in accordance with the procedure of Example 1 was heat treated using a Kason Fluidized bed equipment (K30/40-1FBD-SS Vibratory Fluid Bed Dryer - 30 inch). The sample was heat treated at 3 different temperatures 105°C, 115°C and 130°C. The time of heat treatment was varied from 0-120 minutes for each of the 3 temperatures. Each heat treated sample and control sample was then formulated into a gel according to the following procedure. First 98.2 grams of water was added to a 150ml beaker and mixed using a jiffy mixer blade with a vortex extending to the bottom of the beaker. Next 1 gram of the heat treated powder (above) was slowly added to the vortexing water. This was allowed to mix until homogenous. Once this was homogenous -0.30 grams of 99% Triethanolamine (Fisher Chemical, Fair Lawn, NJ) was added. This was mixed until homogenous and then 0.5 grams of Glydant Plus® (DMDM Hydantoin and lodopropynyl Butylcarbamate) 1,3-Dihydroxylmethyl-5, 5-Dimethylhydantoin and 3-lodo-2-Propynyl Butyl Carbamate (LONZA Corporation, Allendale, NJ) was added. The mixture was placed into a screw top jar and then centrifuged at 2500 rpm for 10 minutes. Viscosity was then measured using a Brookfield Viscometer RVDV-1 PRIME with spindle TC / 10 RPM / 1 minute with samples at 25°C. The results as illustrated in Figure 2 show that higher temperatures and longer heat treatment times provide higher viscosities, with the samples heated to 130°C and 115°C delivering higher viscosities than the samples heated to 105°C.

### Example 42:

740.5 grams of water was charged into a reactor and heated to 38°C. Next, 64.14 grams of 92.5% Star DRI 5 were charged into a reactor. The reactor was then heated to 81°C. An overhead nitrogen sparge was started upon initial heating of the water. The starch solution was cooked for 60 minutes at 81°C. A monomer solution containing 126.1 grams ethyl acrylate and 55.7 grams methacrylic acid was subsequently added to the reactor over a period of 90 minutes. An initiator solution containing 0.69 gram of ammonium persulfate and 78.2 grams water was added to the reactor at the same time as the monomer solutions over a period of 90 minutes. The reaction product was held at 81°C for an additional 120 minutes. The final product was a homogeneous, white emulsion with a total solids content of 22.5%. The resulting emulsion was spray dried to produce a white non-tacky powder.

### Example 43:

722.5 grams of water was charged into a reactor and heated to 38°C. Next, 49.8 grams of 92.5% Star DRI 5 and 16.4 grams of 93.6% Star DRI 1 were charged into a reactor. The reactor was then heated to 81°C. An overhead nitrogen sparge was started upon initial heating of the water. The starch solution was cooked for 60 minutes at 81°C. A monomer solution containing 127.9 grams ethyl acrylate and 56.5 grams methacrylic acid was subsequently added to the reactor over a period of 90 minutes. An initiator solution containing 0.70 gram of ammonium persulfate and 79.2 grams water was added to the reactor at the same time as the monomer solutions over a period of 90 minutes. The reaction product was held at 81°C for an additional 120 minutes. The final product was a homogeneous, white emulsion with a total solids content of 23.1%. The resulting emulsion was spray dried to produce a white non-tacky powder.

### Example 44:

755.5 grams of water was charged into a reactor and heated to 38°C. Next, 33.2 grams of 92.5% Star DRI 5 (DE 5 waxy maltodextrin from Tate and Lyle) and 32.8 grams of 93.6% Star DRI 1 (DE 1 waxy maltodextrin from Tate and Lyle) were charged into a reactor. The reactor was then heated to 81°C. An overhead nitrogen sparge was started upon initial heating of the water. The starch solution was cooked for 60 minutes at 81°C. A monomer solution containing 127.9 grams ethyl acrylate and 56.5 grams methacrylic acid was subsequently added to the reactor over a period of 90 minutes. An initiator solution containing 0.70 gram of ammonium persulfate and 79.2 grams water was added to the reactor at the same time as the monomer solutions over a period of 90 minutes. The reaction product was held at 81°C for an additional 120 minutes. The final product was a homogeneous, white emulsion with a total solids content of 22.8%. The resulting emulsion was spray dried to produce a white non-tacky powder.

### Example 45: Evaluation in Surfactant Formulations - at low pH

Formulations were prepared with the rheology modifiers of the present invention and surfactants and other additives commonly used in personal care cleansing formulations such as shampoos and body washes made in accordance with the following procedure. The rheology modifiers used were those produced in Examples 42, 43, and 44, with no additional heat treatment

A glass beaker was obtained and 39.51 grams of water was added. This was placed under an overhead mixer using a 1.5 inch Jiffy mixing blade. Next 0.3 grams of the powder sample from Example 42 or Example 43 or Example 44 was slowly added to the 39.51 grams of water that was mixing with a vortex extending to the bottom of the beaker. This was mixed until it was uniform. Next 50 grams of Standapol® ES-2 Sodium Lauryl Ether Sulfate (Cognis Corporation, Ambler, PA) (24.8% active) was added and mixed until uniform. Then 0.44 grams of 25% Sodium Hydroxide solution (JT Baker, Center Valley, PA) was added and mixed until uniform. This was followed by adding 7.4 grams of Croderteric™ Cocamidoproply betain (Croda USA, Edison, NJ) (35.1% active) and mixed until uniform. Then 0.05 grams of Dissolvine® 220S Tetra Sodium EDTA (AkzoNobel, Chicago IL) was added and mixed until uniform. This was followed by 0.25 grams of Sodium Benzoate (Spectrum Chemical, New Brunswick, NJ) and mixed until uniform. Then 1.1 grams of 50% Citric acid (Fisher Chemical, Fair Lawn, NJ) was added to adjust the pH to ∼5.0 +/- 0.5. The viscosity of this formulation was then measured using a Brookfield viscometer. The clarity of the sample was also measured. Next 1.0 gram of Floraspheres® JoJoba MDS tequila Sunrise Beads (Floratech, Chandler, AZ) were added to test the suspension properties of the experimental polymers. The beads were evenly mixed with the sample until uniform. Bead suspension was tested by placing the samples in a 45°C oven and visually inspecting the samples for bead migration. Suspension was considered acceptable if the beads showed no migration. If the beads showed movement suspension was considered unacceptable. The results are shown in Table VI.

**Table VI**

| **Sample** | **Turbidity NTU** | **pH** | **Viscosity cps RV#5 @ 10 rpm** | **Suspension of Floratech Beads at 45°C/ 30 days** |
|---|---|---|---|---|
| No Polymer | | 5.1 | 200 | No |
| Example 42 | 11.1 | 5.1 | 5800 | No |
| Example 43 | 18.2 | 5.17 | 11400 | Yes |
| Example 44 | 25.6 | 5.15 | 10920 | Yes |

All of the samples delivered a significant increase in viscosity. However, the samples with the higher levels of the higher molecular weight starch (DE 1) gave suspension in addition to a boost in viscosity.

### Example 46:

740.6 grams of water was charged into a reactor and heated to 38°C. At 38°C, 67.4 grams of 93.4% Star DRI 1 were charged into a reactor. The reactor was then heated to 81°C. An overhead nitrogen sparge was started upon initial heating of the water. The starch solution was cooked for 60 minutes at 81°C. A monomer solution containing 131.1 grams ethyl acrylate and 57.9 grams methacrylic acid was subsequently added to the reactor over a period of 90 minutes. An initiator solution containing 0.72 gram of ammonium persulfate and 81.2 grams water was added to the reactor at the same time as the monomer solutions over a period of 90 minutes. The reaction product was held at 81°C for an additional 120 minutes. The final product was a homogeneous, white emulsion with a total solids content of 22.9%. The resulting emulsion was spray dried to produce a white non-tacky powder.

### Example 47: Evaluation in Surfactant Formulations - low pH

A rheology modifier of the present invention was evaluated in a common body wash/shampoo formulation using ammonium lauryl ether sulfate.

A glass beaker was obtained and 73.86 grams of water was added. This was placed under an overhead mixer using a 1.5 inch Jiffy mixing blade. Next 0.15 grams of Dissolvine® 220S tetra Sodium EDTA (AkzoNobel, Chicago IL) was added to the 73.86 grams of water that was mixing with a vortex extending to the bottom of the beaker. This was mixed until it was uniform. Then 5.25 grams of the polymer of Example 46 was added and mixed until uniform. Next 59 grams of Standapol® EA-2 Ammonium Lauryl Ether Sulfate (Cognis Corporation, Ambler, PA) (24.8% active) was added and mixed until uniform. Next 1.77 grams of 25% Sodium Hydroxide solution (JT Baker, Center Valley, PA) was added and mixed until uniform. This was followed by adding 8.97 grams of Croderteric™ Cocamidoproply betain (Croda USA, Edison, NJ) (35.1% active) and mixed until uniform. This was followed by 1 gram of Euxyl® K712 Potassium (E,E) hexa-2,4,Dienoate (Schulke and Mayr, Mt. Arlington, NJ) and mixed until uniform. Then 1.1 grams of 50% Citric acid (Fisher Chemical, Fair Lawn, NJ) was added to adjust the pH to ∼5.0 +/- 0.5. Next 1.0 gram of Floraspheres® JoJoba MDS tequila Sunrise Beads (Floratech, Chandler, AZ) were added to test the suspension properties of the experimental rheology modifiers. The beads were evenly mixed with the sample until uniform. Bead suspension was tested by placing the samples in a 45°C oven and visually inspecting the samples for bead migration. Suspension was considered acceptable if the beads showed no migration. If the beads showed movement suspension was considered unacceptable.

The results are presented in Table VII.

**Table VII**

| **Sample** | **Turbidity NTU** | **pH** | **Viscosity cps RV#5 @ 10 rpm** | **Suspension of Floratech Beads at 45°C/ 30 days** |
|---|---|---|---|---|
| Surfactant Control with no rheology modifier | 2.83 | 5.23 | 20 | No |
| Example 46 | 56.6 | 5.05 | 11,020 | Yes |

### Example 48: Evaluation in Surfactant Formulations - low pH

A glass beaker was obtained and 162.56 grams of water was added. This was placed under an overhead mixer using a 1.5 inch Jiffy mixing blade. Next 1.4 grams of sample of Example 43 was slowly added to the 162.56 grams of water that was mixing with a vortex extending to the bottom of the beaker. This was mixed until it was uniform. Then 125.48 grams of Standapol® ES-2 Sodium Lauryl Ether Sulfate (Cognis Corporation, Ambler, PA) (24.8% active) was added and mixed until uniform. Next 54.28 grams of Stanfax-234 Sodium Lauryl Sulfate (Para-Chem®, Dalton, GA) was added and mixed until uniform. Then 2.08 grams of 25% Sodium Hydroxide solution (JT Baker, Center Valley, PA) was added and mixed until uniform. This was followed by adding 45.6 grams of Croderteric™ Cocamidopropyl betaine (Croda USA, Edison, NJ) (35.1% active) and mixed until uniform. Then 4.0 grams Glydant Plus® (DMDM Hydantoin and lodopropynyl Butylcarbamate) 1,3-Dihydroxylmethyl-5, 5-Dimethylhydantoin and 3-lodo-2-Propynyl Butyl Carbamate (LONZA Corporation, Allendale, NJ) was added to the solution and mixed minutes until clear and uniform. Next 1.0 gram of Floraspheres® JoJoba MDS tequila Sunrise Beads (Floratech, Chandler, AZ) were added to test the suspension properties of the experimental polymers. The beads were evenly mixed with the sample until uniform. Bead suspension was tested by placing the samples in a 45°C oven and visually inspecting the samples for bead migration. Suspension was considered acceptable if the beads showed no migration. If the beads showed movement suspension was considered unacceptable. The results are shown in Table VIII.

**Table VIII**

| **Sample** | **Turbidity NTU** | **pH** | **Viscosity cps RV#5 @ 10 rpm** | **Suspension of Floratech Beads at 45°C/ 30 days** |
|---|---|---|---|---|
| Surfactant Control with no Polymer | 3.45 | 5.18 | 8,300 | No |
| Example 47 | 16.5 | 5.05 | 30,500 | Yes |

### Example 49: Polysaccharide present during polymerization reaction

780 grams of water and 57.9 g of 80.9 % PR1004B potato starch (from Avebe) were charged into a reactor and heated to 82°C. An overhead nitrogen sparge was started upon initial heating of the water. The starch was cooked for 60 minutes at 82°C. A monomer solution containing 70 grams ethyl acrylate, 50.7 grams methacrylic acid, 17.52 grams methyl methacrylate, and 0.5518 grams trimethylolpropane triacrylate was subsequently added to the reactor over a period of 90 minutes. At the end of the 90 minutes, 5 grams of rinse water was added to the reactor through the same feed tube as fed the monomer solution. An initiator solution containing 0.58 gram of ammonium persulfate and 58.1 grams water was added to the reactor at the same time as the monomer solution over a period of 90 minutes. The reaction product was held at 82°C for an additional 120 minutes. The reactor contents were allowed to cool to ambient temperature. 8.25 g of 10% sodium hydroxide solution was added over a period of 20 minutes. The sample continued to mix for 30 minutes, then 52 grams of 10% sodium bisulfite solution was added over 60 minutes. 1 gram of Proxel GXL was added to the reactor contents. The final product was a homogeneous, white emulsion with a total solids content of around 16.5%. This reaction product was then spray dried to produce a non-tacky white powder with an average particle size of around 10-20 microns. 20 grams of the spray dried sample above was heated in a vented oven at 130°C for a period 60 minutes.

### Example 50: Polysaccharide present during polymerization reaction

780 grams of water and 57.9 g of 80.9 % PR1004B potato starch (from Avebe) were charged into a reactor and heated to 82°C. An overhead nitrogen sparge was started upon initial heating of the water. The starch was cooked for 60 minutes at 82°C. A monomer solution containing 78.8 grams ethyl acrylate, 50.7 grams methacrylic acid, 8.76 grams methyl methacrylate, and 0.5518 grams trimethylolpropane triacrylate was subsequently added to the reactor over a period of 90 minutes. At the end of the 90 minutes, 5 grams of rinse water was added to the reactor through the same feed tube as fed the monomer solution. An initiator solution containing 0.58 gram of ammonium persulfate and 58.1 grams water was added to the reactor at the same time as the monomer solution over a period of 90 minutes. The reaction product was held at 82°C for an additional 120 minutes. The reactor contents were allowed to cool to ambient temperature. 8.25 g of 10% sodium hydroxide solution was added over a period of 20 minutes. The sample continued to mix for 30 minutes, then 52 grams of 10% sodium bisulfite solution was added over 60 minutes. 1 gram of Proxel GXL was added to the reactor contents. The final product was a homogeneous, white emulsion with a total solids content of around 16.1%. This reaction product was then spray dried to produce a non-tacky white powder with an average particle size of around 10-20 microns. 20 grams of the spray dried sample above was heated in a vented oven at 130°C for a period 60 minutes.

### Example 51: Comparison with formulations made with CARBOPOL® 980

Formulations were prepared using the heat treated rheology modifiers of Examples 49 and 50 using the following procedure: Into the main beaker 95.93 grams of water was added and mixed using an overhead mixer fitted with a 1.5 inch Jiffy mixer blade with a vortex extending to the bottom of the beaker. Next 3.5 grams of the sample rheology modifier was slowly added to the water mixing with a vortex extending to the bottom of the beaker. This was allowed to mix for 30 minutes until uniform. Then 0.57 grams of Triethanolamine (Fisher Chemical, Fair Lawn, NJ) was added and mixed until uniform. Once the batch was uniform it was transferred to screw top jars and centrifuged.

The formulation samples were evaluated for turbidity, pH and viscosity. The results are presented in Table IX.

**Table IX**

| **Sample** | **Heat Treatment 130°C time in minutes*** | **Turbidity NTU** | **pH** | **Viscosity cps TC/@10 rpm** |
|---|---|---|---|---|
| Example 49 | 60 | 188 | 6.8 | 37800 |
| Example 50 | 60 | 161 | 6.8 | 18500 |

| | | | | |
|---|---|---|---|---|
| *Heat treatment as described in Example 8 | | | | |

### Example 52: Subjective Hair evaluations

Subjective hair evaluations were carried out. The hair used in these evaluations was 10" long, 2-gram swatches of European virgin brown hair. 5 grams of hair gel was applied on each of the clean, wet hair swatches. The gel was worked into the swatch with 10 downward strokes, then combed through once on each side and dried for one hour at 120°F. The swatches were evaluated for Gloss, Stiffness, Dry comb, Flake, Anti-Static and Feel, defined in Table X below. Four trained panelists each evaluated two pairs of treated hair swatches; one control and one test product in each pair. The swatches were numbered so that the panelist performing the test did not know the identity of the products being tested. For each performance parameter, the panelist was required to select one swatch as better than the other. Equal ratings were not permitted. These tests demonstrated how the products compared in performance. Differences in performance between the two samples were regarded as being statistically significant at the 95% confidence level only if the sample displayed differences in at least seven of the eight comparisons. Samples were rated as "same as control" if they displayed a positive recommendation of less than 7 out of 8 but better that a negative comparison of 7 out of 8. They were rated as "better than control" if they displayed superiority in seven out of eight comparisons. They were rated as a "worse than control" if they displayed a deficiency in seven out of eight comparisons.

**Table X: Hair Subjective Evaluation Parameters**

| **Term** | **Definition** |
|---|---|
| GLOSS | Gently handle the swatches so as not to break the film or stiffness. Visually inspect the swatches to determine which has more shine/gloss. |
| STIFFNESS | Gently handle the swatches and "feel" for differences in Stiffness. Using two fingers, hold the middle of the swatch in a Horizontal position ---- does one bend more than the other? Choose the one that is more rigid. |
| DRY COMBING | Comb each swatch gently five (5) times and evaluate ease of combing. Choose the one that combs more easily. |
| FLAKE | Visually inspect both the swatches after combing. Check the Teeth of the comb for flake accumulation. Holding the swatch at the bound end, run your fingernail down the length of the Tress, then inspect. Choose the one with less flakes. |
| ANTI-STATIC | Holding the swatch by the bound end, comb through vigorously 10 times and evaluate for extent of "flyaways" generated. Choose the swatch with more "flyaways". |
| FEEL | Handle the swatches and determine which one feels more Silky/softer. |

One formulation was prepared using sample of Polymer from Example 39 heat treated at 130°C for 60 minutes.

A comparative sample was prepared with CARBOPOL® 980 according to the following procedure:
**Part A** - 58.0 grams of distilled water was added to a 250 ml beaker and mixed with a vortex extending to the bottom of the beaker. A total of 0.5 g of CARBOPOL® 980 (Lubrizol Advanced Materials Inc, Cleveland, OH) was sifted onto the surface of the mixing water over 5 minutes and was mixed for 20 minutes until uniform. Then 2-Amino-2-Methyl-1 Propanol (Angus Chemical, Buffalo Grove, III) was added to obtain a pH of 6.5 +/- 0.5 as needed (approximately 0.5 g). Next 0.5 grams Glydant Plus® (DMDM Hydantoin and lodopropynyl Butylcarbamate) 1,3-Dihydroxylmethyl-5, 5-Dimethylhydantoin and 3-lodo-2-Propynyl Butyl Carbamate (LONZA Corporation, Allendale, NJ) was added to the solution and mixed for approximately 10 minutes until clear and uniform.
**Part B** - 37.5 grams of water was added to a 150 ml beaker and mixed with good vortexing. This was followed by adding 3.0 grams of polyvinylpyrridone K90 (Ashland Specialty Products, Bridgewater, NJ) and poured into the vortex and mixed for approximately 20 minutes until clear and uniform. Then Part B was slowly added to the main beaker (Part A). The completed gel was slowly mixed (overhead mixer at approximately 50 rpm) for an additional 10 minutes until uniform.

The results are shown in Table XI.

**Table XI**

| **Term** | **0.5% CARBOPOL® 980 / 3.0% PVP K90** | **3.5% Polymer of Example 39 (60 minutes of heat treatment)** | |
|---|---|---|---|
| GLOSS | 1 | 7 | Better than control |
| STIFFNESS | 0 | 8 | Better than control |
| DRY COMBING | 2 | 6 | Same as control |
| FLAKE | 6 | 2 | Same as control |
| ANTI-STATIC | 5 | 3 | Same as control |
| FEEL | 3 | 5 | Same as control |

The rheology modifier of the present invention provided hair gel subjective properties with superior Gloss and Stiffness when compared to common CARBOPOL® 980/PVP K90 hair gels. No negative effects were noted on the Dry Comb, Flake, Anti-Static, or Feel properties.

### Example 53: High Humidity Curl Retention

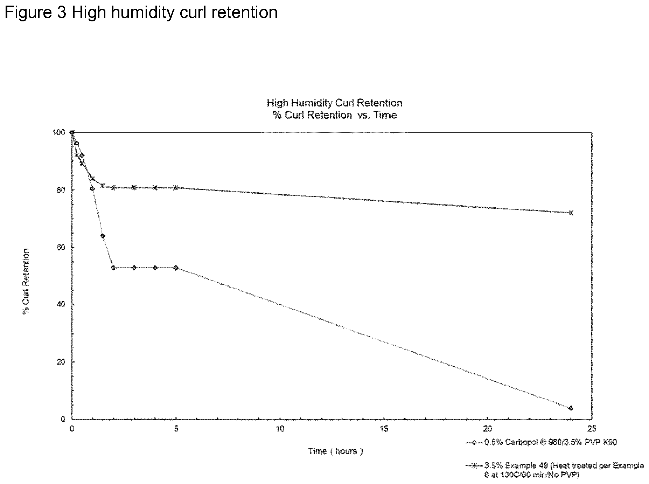

The same two formulations that were compared in Example 52 were evaluated with respect to high humidity curl retention. These evaluations used hair swatches with a fixed amount of hair gel applied on clean, wet 10" long, 2-gram swatches of European virgin brown hair, the sample was worked into the swatch and combed through. Each hair tress was then rolled into a curl with the use of a plastic mandrel. The mandrels were carefully removed and the curls were secured with two common hair clips. The curls were then allowed to dry in a 120°F oven over night. The curls were removed from the oven and placed on Plexiglas boards with a fixed measuring scale. The curls were gently unwound with a glass rod. Initial curl height readings were taken and the boards were placed into a controlled humidity cabinet set at 70°F/90% relative humidity. The curl lengths were measured at 15 minutes, 30 minutes, 60 minutes, 90, minutes, 2 hours, 3 hours, 4 hours, 5 hours and 24 hour time intervals. The measured curl heights were used to calculate the % curl retention values. The results are illustrated in Figure 3. The rheology modifier of the present invention provided high humidity curl retention of about 80% after 24 hours, while the control sample provided almost no curl retention after 24 hours.

### Example 54: Skin Care formulations

Formulations suitable for use as skin care compositions can be prepared by the following procedure.

Place 78.46 grams of water into a beaker. Mix this with a 1.5" jiffy mixer blade with a vortex extending to the bottom of the beaker. Then add 3.5 grams of Polymer of Example 1. Mix this until uniform and homogenous. Start heating to 75°-80°C. When temperature is reached begin adding 0.67 grams of D Panthenol 75L (DSM Nutritional). Mix until uniform and homogenous. Hold this at 75°-80°C with good mixing. In beaker B add 6.0 grams of Cutina® GMS-SE, Glycerol Stearate SE ( BASF Corporation), Then add 2.0 grams Lanette® O Cetearyl Alcohol (BASF Corporation). Then add 2.0 grams of Pristerene™ 4916-SO-(GD) fatty acids C16-C18 (Croda Corporation, Edison, NJ). Next add 3.0 grams of Tegosoft® OS Ethylhexyl Stearate (Evonik, Piscataway, NJ). Then add 3.0 grams of Drakeol® 7 LT mineral oil (Penrico, Karns, City PA). Heat this phase to 75°-80°C and mix with a stir bar until homogenous. Once uniform slowly add beaker B to beaker A under homogenization. Once the two beakers are combined stop homogenization after 5 minutes and return to overhead mixing with the 1.5" jiffy mixer blade. Begin cooling and then add 0.57 grams of Triethanolamine (Fisher Chemical, Fair Lawn, NJ). If needed QS pH to 5.5-6.6 using additional Triethanolamine (Fisher Chemical, Fair Lawn, NJ). Once cool to 45°C add 0.80 grams of Euxyl® PE9010 Phenoxyethanol (and) Ethylhexylglycerin (Shulke and Mayr, Mt. Arlington, NJ).

### Example 55

692.7 grams of water was charged into a reactor and heated to 38°C. Next, 18.2.g of 82.3 % Perfectamyl A4692 (potato starch from Avebe) and 47.8 grams of 92.5% Star DRI 5 were charged into a reactor. The reactor was then heated to 81°C. An overhead nitrogen sparge was started upon initial heating of the water. The starch solution was cooked for 60 minutes at 81°C. A monomer solution containing 122.6 grams ethyl acrylate and 54.1 grams methacrylic acid was subsequently added to the reactor over a period of 90 minutes. An initiator solution containing 0.67 gram of ammonium persulfate and 75.9 grams water was added to the reactor at the same time as the monomer solutions over a period of 90 minutes. The reaction product was held at 81°C for an additional 120 minutes. The final product was a homogeneous, white emulsion with a total solids content of 22.6%. This reaction product was then spray dried to produce a non-tacky white powder

### Example 56 Acid swellable reacted with polysaccharide

240.27 grams of DE 10 maltodextrin (92.65%) were dissolved in 726 grams of water in a 2 liter reactor fitted with an agitator, condenser, thermometer and heated to 65°C and sparged with subsurface N₂ for 60 minutes. 6.86 grams of Polystep® TD 507 surfactant was added next. Next 7.0 grams of cetyl 20 EO itaconate associative monomer was added. 1.47 grams of triethanolamine was then added.

The temperature was then lowered to 55°C and 87.7 grams of ethyl acrylate was added over 10 minutes. Then 7.52 grams of propylene glycol was added. Next 49.75 grams of N'N-dimethyl aminoethylmethacrylate was and added to the reactor in 10 minutes. A first initiator solution containing 0.858 grams of sodium persulfate dissolved in 376.49 grams of water was added over 90 minutes. A second initiator solution containing 2.51 grams of sodium bisulfite (41% aqueous solution) dissolved in 380.56 grams of water was added over 90 minutes. The reaction was not allowed to exotherm over 60°C and was cooled if necessary. At the end of these feeds an initiator solution containing 0.53 grams of sodium persulfate dissolved in 15.51 grams of water was added over 60 minutes while holding the temperature at 60°C. The reactor contents were held at 70°C for 1 hour. The emulsion above was spray dried to a white powder.

### Example 57 Acid swellable reacted with polysaccharide

200.51 grams of DE 10 maltodextrin (92.65%) were dissolved in 668.25 grams of water in a 2 liter reactor fitted with an agitator, condenser, and thermometer and heated to 65°C and sparged with subsurface N₂ for 60 minutes. 8.59 grams of Polystep® TD 507 surfactant was added next. Next 8.76 grams of cetyl 20 EO itaconate associative monomer was added. 1.85 grams of triethanolamine was then added. The temperature was then lowered to 55°C and 102.28 grams of ethyl acrylate was added over 10 minutes. Then 9.41 grams of propylene glycol was added. Next 62.29 grams of N'N-dimethyl aminoethylmethacrylate was and added to the reactor in 10 minutes. A first initiator solution containing 1.07 grams of sodium persulfate dissolved in 405.0 grams of water was added over 90 minutes. A second initiator solution containing 3.14 grams of sodium bisulfite (41% aqueous solution) dissolved in 418.82 grams of water was added over 90 minutes. The reaction was not allowed to exotherm over 60°C and was cooled if necessary. At the end of these feeds an initiator solution containing 0.66 grams of sodium persulfate dissolved in 19.42 grams of water was added over 60 minutes while holding the temperature at 60°C. The reactor contents were held at 70°C for 1 hour. The emulsion above was spray dried to a white powder.

### Example 58 Heat treatment and performance of dried rheology modifiers containing acid swellable polymers:

The powder samples of Example 56 and Example 57 (containing 40% and 50% weight percent active acid swellable polymer respectively) were heated in an oven at 130°C in individual samples of 4.5 grams each for 45, 60, and 90 minutes. 1% active solutions in water were then prepared with those samples, including non-heat-treated powders of each as controls:
Testing of the samples from Example 57: 4 gram samples of powder of the polymers from Example 57 heat treated for different times were each slowly dispensed into 196 grams of water with overhead stirring. The samples were stirred for 1.5 hours at 780 rpm. 2 grams of 50% D-Gluconic acid was added via pipette into each of the solutions with constant stirring. The samples were stirred an addition one hour.

Testing of the samples from Example 56: 4 gram samples of powder of the polymers from Example 56 heat treated for different times were each slowly dispensed into 156 grams of water with overhead stirring. The samples were stirred for 1.5 hours at 780 rpm. 1.6 grams of 50% D-Gluconic acid was added via pipette into each of the solutions with constant stirring. The samples were stirred an additional one hour.

Viscosity was measured using a Brookfield DV-1+ viscometer at 10 and 20 rpm with a gauge 6 spindle. The results are shown in Table XIII.

**Table XIII**

| **Sample** | **Heat-treat time** | **Viscosity** | | **pH** |
|---|---|---|---|---|
| | (min) | 10 rpm (cps) | 20 rpm (cps) | |
| Polymer of Example 56 | 0 | 1600 | 950 | 3.45 |
| | 45 | 5000 | 3100 | 3.1 |
| | 60 | 8100 | 4950 | 3.14 |
| | 90 | 8400 | 5250 | 3.2 |
| Polymer of Example 57 | 0 | 2000 | 1350 | 3.4 |
| | 45 | 6700 | 4000 | 3.3 |
| | 60 | 8200 | 4900 | 3.43 |
| | 90 | 7800 | 5000 | 3.28 |

These data indicate that even the non-heat treated sample will develop a significant amount of viscosity when neutralized with an acid to low pH. There is a trend of increasing viscosity with increasing heat-treatment time. Also, the heat treated samples had higher viscosity than the non-heat treated samples. It was observed that the heat-treated samples also exhibited a distinct and pronounced short-flow property.

### Example 59: Blend of acid swellable (Example 31) with starch

6.6 kg of hot tap water was weighed into a 5-gallon bucket and a Heidolph stirrer was set up to agitate the liquid contents of the bucket. The stirrer was fitted with a 2" dispersion blade and set to mix at 1200 rpm. While the water was mixing 4.1 kg of StarDri 10 (DE10 maltodextrin, 91.6% solids) was weighed and gently sifted into the water. The mixture was allowed to stir until all the maltodextrin powder was fully incorporated forming a homogenous solution. To this maltodextrin solution, 6.0 kg of acid swellable polymer (made in accordance with the procedure of Example 31) was added and mixed until homogenous. Once mixed, the solution was fed into a Bowen Tower spray dryer through a Masterflex 7524-50 Pump and into a Dual Fluid Atomization nozzle and then into the Bowen Spray Dryer. The operating parameters of the operation were a pump speed between 50 and 55 mL/min, air atomization pressure of 80psi, and outlet dryer temperature between 88 and 92°C. This process yielded a non-tacky white powder.

### Examples 60-63 - Evaluation of Example 59 in slow set asphalt emulsions

The rheology modifier produced in Example 59 was evaluated in slow set asphalt emulsions. The materials were made according to the formulations shown in Table XIV.

**Table XIV**

| **Formulation** | **Example 60 (comparative)** | **Example 61** | **Example 62** | **Example 63** |
|---|---|---|---|---|
| PG 64-22 bitumen | 60.0 | 60.0 | 60.0 | 60.0 |
| Redicote® E-4868 | 1.5 | 1.5 | 1.5 | 1.5 |
| Example 59 | --- | 0.24 | 0.48 | 0.72 |
| HCl, 37% conc. | 0.19 | 0.22 | 0.22 | 0.23 |
| Water | To 100 | To 100 | To 100 | To 100 |
| pH of soap solution | 2.0 | 2.0 | 2.0 | 2.0 |

The rheology modifier produced in Example 59 was dissolved in warm water, following which the Redicote® E-4868 (AkzoNobel Surface Chemistry LLC, Chicago, IL) emulsifier is added before adding the hydrochloric acid (Sigma-Aldrich Corporation). This soap phase is added to PG64-22 bitumen (Holly Frontier Corporation) and an emulsion is produced using a colloid mill, using a rotor and stator with mill speed of starting at 3600 and increasing up to a maximum of 9000 rpm. Determination of the mill speed is understood by those of ordinary skill in the art.

Samples were tested following manufacture and additionally following 1 week's storage at 55 °C. In each case the samples were allowed to cool to room temperature before testing.

Saybolt Furol seconds (SSF) Viscosity is tested (as per ASTM D7496) by measuring the time taken for 60 cm³ to pass through a Saybolt Furol viscosimeter at a specified temperature. The results shown indicate the temperature used in each test.

Cement mix test is tested (as per ASTM D 6935) by mixing a specified amount of diluted emulsified asphalt and 50 g Type III Portland cement for 1 minute. A further 150 mL of distilled water is then added and mixing continued for a further 3 mins. The mixture is then decanted into a No. 14 mesh sieve and the amount of retained material in the sieve is then calculated. Residue evaporation was tested (as per ASTM D 6934) by taking an accurately weighed sample of approximately 50 g, placing it on a non-flammable substrate and heating it for 3 hours in an oven at 163 °C and then measuring the mass of residual material. The percentage is calculated by taking the mass of residual material and dividing it by the original mass multiplied by 100.

Particle size was measured using a Beckman Coulter LS 13 320, Laser Diffraction Particle size Analyser.

The data initially and after one week are shown in Table XV.

**Table XV**

| **Initial Characterisation** | | | | |
|---|---|---|---|---|
| | **Example 60 (comparative)** | **Example 61** | **Example 62** | **Example 63** |
| Appearance | Smooth, brown | Smooth, brown | Smooth, brown | Smooth, brown |
| Residue by Evap, pct. | 60.2 | 60.5 | 60.7 | 61.1 |
| Sieve, 800µ, pct. | Nil | Nil | Nil | Nil |
| Visc. SSF, 25°C, sec. | 17 | 26 | 36 | 50 |
| Cement Mix Test, pct. | 0.09 | 0.20 | 0.56 | 0.60 |
| Particle size median | 4.763 | 3.921 | 3.905 | 3.749 |
| Particle size mean | 7.439 | 5.820 | 5.847 | 6.239 |

| **After 1 week of storage at 55°C** | | | | |
|---|---|---|---|---|
| Appearance after the emulsion cool down | Water layer on top and slight soft asphalt settled on bottom | Thin layer of water on top and no asphalt settlement | Light creaming or asphalt thickening on top, no asphalt settlement | Heavy creaming or asphalt thickening on top, no asphalt settlement |
| Visc. SSF, 25°C, sec. | 19 | 35 | 51 | 72 |

### Examples 64-67 Evaluation of Example 59 in rapid set asphalt emulsion

The polymer produced in Example 59 was evaluated in rapid set asphalt emulsions. The materials were made according to the formulations shown in Table XVI.

The rheology modifier of Example 59 was dissolved in warm water, following which the Redicote emulsifier is added before adding the hydrochloric acid. This soap phase is added to PG64-22 bitumen and an emulsion is produced using a colloid mill, using a rotor and stator with mill speed of starting at 3600 and increasing up to a maximum of 9000 rpm. Judging the mill speed is something understood by someone of ordinary skill in the art.

**Table XVI**

| | **Example 64 (comparative)** | **Example 65** | **Example 66** | **Example 67** |
|---|---|---|---|---|
| PG 64-22 Bitumen | 60.0 | 60.0 | 60.0 | 60.0 |
| Redicote EM-44A | 0.3 | 0.3 | 0.3 | 0.3 |
| Example X1 | --- | 0..24 | 0.48 | 0.72 |
| HCI, 37% conc. | 0.2 | 0.21 | 0.23 | 0.24 |
| Water | To 100 | To 100 | To 100 | To 100 |
| pH of soap solution | 2.0 | 2.0 | 2.0 | 2.0 |
| | | | | |

| **Initial Characterisation** | | | | |
|---|---|---|---|---|
| Appearance | No creaming, sl. soft ac settlement | No creaming, no settlement | Very light creaming, no settlement | Some creaming, no settlement |
| Residue by Evap, pct. | 60.3 | 60.5 | 60.6 | 60.9 |
| Sieve, 800µ, pct. | Nil | Nil | Nil | Nil |
| Visc. SSF, 25°C, sec. | 17 | 20 | 25 | 29 |
| Visc. SSF, 50°C, sec. | 15 (b) | 17 (b) | 21 (b) | 21 (b) |
| Demulsibility Test, pct | 49.9 | 47.2 | 47.3 | 43.3 |
| Particle size median | 4.905 | 4.541 | 4.386 | 4.236 |
| Particle size mean | 6.792 | 5.973 | 6.678 | 5.947 |

| **After 1 week of storage at 55°C** | | | | |
|---|---|---|---|---|
| Appearance | No creaming, sl. soft ac settlement | No creaming, no settlement | No creaming, no settlement | No creaming, no settlement |
| Visc. SSF, 50°C, sec. | 16 | 16 | 21 | 22 |
| Visc. SSF, 25°C, sec. | 22 | 25 | 29 | 32 |

### Example 68 Blend of acid swellable (Example 31) with starch

6.6 kg of hot tap water was weighed into a 5-gallon bucket and a Heidolph stirrer was set up to agitate the liquid contents of the bucket. The stirrer was fitted with a 2" dispersion blade and set to mix at 1200 rpm. While the water was mixing 4.4 kg of StarDri 10 (DE10 maltodextrin, 91.6% solids) was weighed and gently sifted into the water. The mixture was allowed to stir until all the maltodextrin powder was fully incorporated forming a homogenous solution. To this maltodextrin solution, 4.8 kg of acid swellable polymer (made in accordance with the procedure of Example 31) was added and mixed until homogenous. Once mixed, the solution was fed into a Bowen Tower spray dryer through a Masterflex 7524-50 Pump and into a Dual Fluid Atomization nozzle and then into the Bowen Spray Dryer. The operating parameters of the operation were a pump speed between 50 and 80 mL/min, air atomization pressure of 80psi, and outlet dryer temperature between 85 and 95°C. This process yielded a non-tacky white powder.

### Example 69 Dried acid swellable rheology modifier in fabric conditioners

Liquid Fabric softeners are typically segregated into two classes, regular and concentrated. The formulations for these will typically contain a quaternary ammonium cationic surfactant, some form of fragrance, and a rheology modifier.

Inclusion of the disclosed dried acid swellable rheology modifiers will provide increase in viscosity. The table below XVII illustrate the effect of both dose and pH on a screening formulation.

The formulations 69-1 - 69-8 were made as follows. The dried polymer (in the amount noted in Table XVII) of Example 68 was added to water which had been heated to between 28-35 °C followed by citric acid if present in the formulation. This mixture was subsequently stirred until homogeneous. At this point molten Arquad 2HT75-E (available from AkzoNobel Surface Chemistry LLC) was added. The mixture was then homogenized for approximately 15 minutes.

The viscosities of these fabric softener formulations were measured at 25°C using a RVDV-1 Brookfield viscometer (Brookfield Engineering Middleboro, MA) using the supplied RV2 spindle at 20 rpm. Samples were allowed to equilibrate for 1 minute before a reading was taken. The viscosities were also measured after 24 hours. The results are shown in Table XVII.

**Table XVII: Use of dried acid swellable rheology modifier in fabric softener formulations**

| | **Weight %** | | | | | | | |
|---|---|---|---|---|---|---|---|---|
| **Formulation Number** | **69-1** | **69-3** | **69-5** | **69-7** | **69-2** | **69-4** | **69-6** | **69-8** |
| Arquad 2HT75 E | 2.0 | 2.0 | 2.0 | 2.0 | 2.0 | 2.0 | 2.0 | 2.0 |
| Polymer of Example 68 | 1.0 | 1.5 | 2.5 | 3.0 | 1.0 | 1.5 | 2.5 | 3.0 |
| Citric Acid | - | - | - | - | 0.1 | 0.1 | 0.1 | 0.1 |
| Water | 97.0 | 96.5 | 95.5 | 95.0 | 96.9 | 96.4 | 95.4 | 94.9 |
| pH | 7.40 | 7.15 | 7.18 | 7.04 | 2.95 | 3.10 | 3.64 | 3.91 |
| Viscosity mPas | 719. 8 | 688. 4 | 574. 4 | 481. 4 | 1782. 0 | 2268. 0 | 4049. 0 | 6761. 0 |
| Viscosity mPas after 24 hrs | 866. 8 | 832. 3 | 842. 8 | 752. 8 | 1344. 0 | 1674. 0 | 2753. 0 | - |

These data indicate that the acid swellable polymers viscosify the fabric softener formulations after the pH is lowered to activate the polymer. This can be seen by comparing the viscosity of formulation 69-1 and 69-2, 69-3 and 69-4, 69-5 and 69-6 and 69-7 and 69-8. These data also illustrate that increasing the polymer concentration increases the viscosity.

The foregoing examples are presented by way of illustration and not by way of limitation. Those skilled in the art will understand other examples and embodiments are encompassed within the present invention. The spirit and scope of the present invention are to be limited only by the terms of any claims presented hereunder.

## Claims

1. A dried rheology modifier composition comprising at least one swellable emulsion polymer and at least one water soluble support polymer, wherein the at least one swellable emulsion polymer comprises at least one ethylenically unsaturated water soluble monomer and at least one ethylenically unsaturated hydrophobic monomer, wherein said dried rheology modifier composition comprises less than 25 wt% water.

2. The dried rheology modifier composition of claim 1 wherein the at least one swellable emulsion polymer is selected from the group consisting of an alkali swellable emulsion polymer and an acid swellable emulsion polymer.

3. The dried rheology modifier composition of any of claims 1-2 wherein the at least one swellable emulsion polymer further comprises an associative monomer.

4. The dried rheology modifier composition of any of claims 1-3 wherein the water soluble support polymer is selected from a polysaccharide, a polysaccharide derivative, and a water soluble polyvinylacetate derivative.

5. The dried rheology modifier composition of any of claims 1-4 wherein the at least one swellable emulsion polymer is surfactant stabilized.

6. The dried rheology composition of any of claims 1-5 further comprising a second composition of a swellable emulsion polymer and a water soluble support polymer, wherein each of the swellable emulsion polymer and the water soluble support polymer of the second composition can be the same as or different from the swellable emulsion polymer and water soluble protective polymer, respectively, of the initial composition.

7. A method of making the dried rheology modifier composition of any of claims 1-6, the method comprising the steps of (i) providing a mixture of monomers comprising at least one ethylenically unsaturated water soluble monomer and at least one ethylenically unsaturated hydrophobic monomer, and a water soluble support polymer, (ii) reacting the monomers that make up the swellable emulsion polymer in the presence of the water soluble support polymer to produce a swellable emulsion polymer composition, said reaction being optionally conducted in the presence of a surfactant, and (iii) drying said swellable emulsion polymer composition.

8. A method of making the dried rheology modifier composition of any of claims 1-6, the method comprising the steps of (i) providing a surfactant stabilized swellable emulsion polymer, (ii) blending the swellable emulsion polymer with a water soluble support polymer, and (iii) drying the blend.

9. The method of any of claims 7-8 wherein said emulsion polymer is an alkali swellable emulsion polymer.

10. The method of any of claims 7-8 wherein said emulsion polymer is an acid swellable emulsion polymer.

11. The method of any of claims 7-10 wherein after the drying step the composition is subjected to a further heat treatment.

12. The method of any of claims 7-11 wherein prior to the drying step a second composition is added, the second composition comprising a second swellable emulsion polymer and optionally a second water soluble protective polymer, wherein each of the second swellable emulsion polymer and the optional second water soluble protective polymer of the second composition can be the same as or different from the at least one swellable emulsion polymer and at least one water soluble protective polymer, respectively, of the initial polymer composition or blend.

13. A product formulation comprising the dried rheology modifier composition of any of claims 1-6 and a product active ingredient, said product formulation being selected from the group consisting of a personal care product formulation, a home care product formulation, a healthcare product formulation, an institutional and industrial care product formulation, an adhesive, an agricultural product formulation, an oil field fracturing product formulation, an asphalt product formulation, a paint formulation, and a water-based protective coating product formulation.

14. The product formulation of claim 13 wherein the formulation is a personal care formulation selected from the group consisting of a liquid cleanser, a body wash, a shampoo, a hair styling product, a skin lotion, a hand sanitizer, and a sun care formulation.

15. A method of making a formulation of any of claims 13-14, the method comprising the steps of (a) providing a dried rheology modifier composition of any of claims 1-6 wherein the swellable emulsion polymer is an alkali swellable emulsion polymer, (b) mixing the dried rheology modifier composition into an aqueous formulation containing surfactants, (c) neutralizing the aqueous formulation to a pH of about 6.5 or higher, and (d) acidifying the aqueous formulation to a pH in the range of about 3 - 6.5.

## Patentansprüche

1. Getrocknete Rheologiemodifizierer-Zusammensetzung, umfassend wenigstens ein quellfähiges Emulsionspolymer und wenigstens ein wasserlösliches Trägerpolymer, wobei das wenigstens eine quellfähige Emulsionspolymer wenigstens ein ethylentisch ungesättigtes, wasserlösliches Monomer und wenigstens ein ethylenisch ungesättigtes, hydrophobes Monomer umfasst, wobei die getrocknete Rheologiemodifizierer-Zusammensetzung weniger als 25 Gew.% Wasser umfasst.

2. Getrocknete Rheologiemodifizierer-Zusammensetzung nach Anspruch 1, wobei das wenigstens eine quellfähige Emulsionspolymer ausgewählt ist aus der Gruppe, bestehend aus einem alkaliquellfähigen Emulsionspolymer und einem säurequellfähigen Emulsionspolymer.

3. Getrocknete Rheologiemodifizierer-Zusammensetzung nach einem der Ansprüche 1-2, wobei das wenigstens eine quellfähige Emulsionspolymer weiterhin ein assoziatives Monomer umfasst.

4. Getrocknete Rheologiemodifizierer-Zusammensetzung nach einem der Ansprüche 1-3, wobei das wasserlösliche Trägerpolymer ausgewählt ist aus einem Polysaccharid, einem Polysaccharidderivat und einem wasserlöslichen Polyvinylacetatderivat.

5. Getrocknete Rheologiemodifizierer-Zusammensetzung nach einem der Ansprüche 1-4, wobei das wenigstens eine quellfähige Emulsionspolymer tensidstabilisiert ist.

6. Getrocknete Rheologiemodifizierer-Zusammensetzung nach einem der Ansprüche 1-5, weiterhin umfassend eine zweite Zusammensetzung eines quellfähigen Emulsionspolymers und eines wasserlöslichen Trägerpolymers, wobei jedes des quellfähigen Emulsionspolymers und des wasserlöslichen Trägerpolymers der zweiten Zusammensetzung das gleiche wie oder ein anderes sein kann als das quellfähige Emulsionspolymer bzw. das wasserlösliche Schutzpolymer der ersten Zusammensetzung.

7. Verfahren zur Herstellung der getrockneten Rheologiemodifizierer-Zusammensetzung nach einem der Ansprüche 1-6, wobei das Verfahren die folgenden Schritte umfasst: (i) Bereitstellen einer Mischung von Monomeren, umfassend wenigstens ein ethylenisch ungesättigtes, wasserlösliches Monomer und wenigstens ein ethylenisch ungesättigtes, hydrophobes Monomer und ein wasserlösliches Trägerpolymer, (ii) Reagierenlassen der Monomere, die das quellfähige Emulsionspolymer bilden, in Gegenwart des wasserlöslichen Trägerpolymers, um eine quellfähige Emulsionspolymer-Zusammensetzung herzustellen, wobei die Reaktion wahlweise in Gegenwart eines Tensids durchgeführt wird, und (iii) Trocknen der quellfähigen Emulsionspolymer-Zusammensetzung.

8. Verfahren zur Herstellung der getrockneten Rheologiemodifizierer-Zusammensetzung nach einem der Ansprüche 1-6, wobei das Verfahren die folgenden Schritte umfasst: (i) Bereitstellen eines tensidstabilisierten, quellfähigen Emulsionspolymers, (ii) Mischen des quellfähigen Emulsionspolymers mit einem wasserlöslichen Trägerpolymer und (iii) Trocknen der Mischung.

9. Verfahren nach einem der Ansprüche 7-8, wobei das Emulsionspolymer ein alkaliquellfähiges Emulsionspolymer ist.

10. Verfahren nach einem der Ansprüche 7-8, wobei das Emulsionspolymer ein säurequellfähiges Emulsionspolymer ist.

11. Verfahren nach einem der Ansprüche 7-10, wobei die Zusammensetzung nach dem Trocknungsschritt einer weiteren Wärmebehandlung unterzogen wird.

12. Verfahren nach einem der Ansprüche 7-11, wobei vor dem Trocknungsschritt eine zweite Zusammensetzung zugefügt wird, wobei die zweite Zusammensetzung ein zweites quellfähiges Emulsionspolymer und wahlweise ein zweites wasserlösliches Schutzpolymer umfasst, wobei jedes des zweiten quellfähigen Emulsionspolymers und des wahlweisen zweiten wasserlöslichen Schutzpolymers der zweiten Zusammensetzung das gleiche wie oder ein anderes sein kann als das wenigstens eine quellfähige Emulsionspolymer bzw. das wenigstens eine wasserlösliche Schutzpolymer der ersten Polymerzusammensetzung oder Mischung.

13. Produktformulierung, umfassend die getrocknete Rheologiemodifizierer-Zusammensetzung nach einem der Ansprüche 1-6, und einen Produktwirkstoff, wobei die Produktformulierung ausgewählt ist aus der Gruppe, bestehend aus einer Körperpflegeproduktformulierung, einer Reinigungsmittelformulierung, einer Gesundheitsproduktformulierung, einer Formulierung für ein institutionelles Pflegeprodukt und ein Industriereinigungsprodukt, einem Klebemittel, einer landwirtschaftlichen Produktformulierung, einer Bohrloch-Fracturing-Produktformulierung, einer Asphalt-Produktformulierung, einer Lackformulierung und einer wasserbasierten Schutzschicht-Produktformulierung.

14. Produktformulierung nach Anspruch 13, wobei die Formulierung eine Körperpflegeproduktformulierung ist, ausgewählt aus der Gruppe, bestehend aus einem flüssigen Reinigungsmittel, einer Körperwaschflüssigkeit, einem Shampoo, einem Haarstylingprodukt, einer Hautlotion, einem Handdesinfektionsmittel und einer Sonnenpflegeformulierung.

15. Verfahren zur Herstellung einer Formulierung nach einem der Ansprüche 13-14, wobei das Verfahren die folgenden Schritte umfasst: (a) Bereitstellen einer getrockneten Rheologiemodifizierer-Zusammensetzung nach einem der Ansprüche 1-6, wobei das quellfähige Emulsionspolymer ein alkaliquellfähiges Emulsionspolymer ist, (b) Einmischen der getrockneten Rheologiemodifizierer-Zusammensetzung in eine wässrige, Tenside enthaltende Formulierung, (c) Neutralisieren der wässrigen Formulierung auf einen pH-Wert von etwa 6,5 oder höher und (d) Ansäuern der wässrigen Formulierung auf einen pH-Wert in dem Bereich von etwa 3-6,5.

## Revendications

1. Composition de modificateur de rhéologie séchée comprenant au moins un polymère en émulsion pouvant gonfler et au moins un polymère de support hydrosoluble, où l'au moins un polymère en émulsion pouvant gonfler comprend au moins un monomère hydrosoluble à insaturation éthylénique et au moins un monomère hydrophobe à insaturation éthylénique, où ladite composition de modificateur de rhéologie séchée comprend moins de 25% en poids d'eau.

2. Composition de modificateur de rhéologie séchée de la revendication 1, dans laquelle l'au moins un polymère en émulsion pouvant gonfler est choisi dans le groupe constitué d'un polymère en émulsion pouvant gonfler en milieu alcalin et d'un polymère en émulsion pouvant gonfler en milieu acide.

3. Composition de modificateur de rhéologie séchée de l'une des revendications 1 et 2, dans laquelle l'au moins un polymère en émulsion pouvant gonfler comprend en outre un monomère associatif.

4. Composition de modificateur de rhéologie séchée de l'une des revendications 1 à 3, dans laquelle le polymère de support hydrosoluble est choisi entre un polysaccharide, un dérivé de polysaccharide et un dérivé d'acétate de polyvinyle hydrosoluble.

5. Composition de modificateur de rhéologie séchée de l'une des revendications 1 à 4, dans laquelle l'au moins un polymère en émulsion pouvant gonfler est stabilisé par un tensioactif.

6. Composition de modificateur de rhéologie séchée de l'une des revendications 1 à 5, comprenant en outre une deuxième composition d'un polymère en émulsion pouvant gonfler et d'un polymère de support hydrosoluble, où chacun du polymère en émulsion pouvant gonfler et du polymère de support hydrosoluble de la deuxième composition peut être identique ou différent du polymère en émulsion pouvant gonfler et du polymère protecteur hydrosoluble, respectivement, de la composition initiale.

7. Procédé de fabrication de la composition de modificateur de rhéologie séchée de l'une des revendications 1 à 6, le procédé comprenant les étapes qui consistent : (i) à fournir un mélange de monomères comprenant au moins un monomère hydrosoluble à insaturation éthylénique et au moins un monomère hydrophobe à insaturation éthylénique et un polymère de support hydrosoluble, (ii) à faire réagir les monomères qui constituent le polymère en émulsion pouvant gonfler en présence du polymère de support hydrosoluble pour produire une composition de polymère en émulsion pouvant gonfler, ladite réaction étant facultativement réalisée en présence d'un tensioactif, et (iii) à sécher ladite composition de polymère en émulsion pouvant gonfler.

8. Procédé de fabrication de la composition de modificateur de rhéologie séchée de l'une des revendications 1 à 6, le procédé comprenant les étapes qui consistent : (i) à fournir un polymère en émulsion pouvant gonfler stabilisé par un tensioactif, (ii) à mélanger le polymère en émulsion pouvant gonfler avec un polymère de support hydrosoluble et (iii) à sécher le mélange.

9. Procédé de l'une des revendications 7 et 8, dans lequel ledit polymère en émulsion est un polymère en émulsion pouvant gonfler en milieu alcalin.

10. Procédé de l'une des revendications 7 et 8, dans lequel ledit polymère en émulsion est un polymère en émulsion pouvant gonfler en milieu acide.

11. Procédé de l'une des revendications 7 à 10, dans lequel, après l'étape de séchage, la composition est soumise à un traitement thermique supplémentaire.

12. Procédé de l'une des revendications 7 à 11, dans lequel, avant l'étape de séchage, une deuxième composition est ajoutée, la deuxième composition comprenant un deuxième polymère en émulsion pouvant gonfler et facultativement un deuxième polymère protecteur hydrosoluble, où chacun du deuxième polymère en émulsion pouvant gonfler et du deuxième polymère protecteur hydrosoluble facultatif de la deuxième composition peut être identique ou différent de l'au moins un polymère en émulsion pouvant gonfler et de l'au moins un polymère protecteur hydrosoluble, respectivement, du mélange ou de la composition initial(e) de polymère.

13. Formulation de produit comprenant la composition de modificateur de rhéologie séchée de l'une des revendications 1 à 6 et un principe actif de produit, ladite formulation de produit étant choisie dans le groupe constitué d'une formulation de produit de soins personnels, d'une formulation de produit de soins à domicile, d'une formulation de produit de soins de santé, d'une formulation de produit de soins institutionnels et industriels, d'un adhésif, d'une formulation de produit agricole, d'une formulation de produit de fracturation de champ pétrolier, d'une formulation de produit d'asphalte, d'une formulation de peinture et d'une formulation de produit de revêtement de protection à base d'eau.

14. Formulation de produit de la revendication 13, dans laquelle la formulation est une formulation de soins personnels choisie dans le groupe constitué d'un nettoyant liquide, d'un nettoyant pour le corps, d'un shampooing, d'un produit de coiffage, d'une lotion pour la peau, d'un désinfectant pour les mains et d'une formulation de protection solaire.

15. Procédé de fabrication d'une formulation de l'une des revendications 13 et 14, le procédé comprenant les étapes qui consistent : (a) à fournir une composition de modificateur de rhéologie séchée de l'une des revendications 1 à 6, où le polymère en émulsion pouvant gonfler est un polymère en émulsion pouvant gonfler en milieu alcalin, (b) à mélanger la composition de modificateur de rhéologie séchée dans une formulation aqueuse contenant des tensioactifs, (c) à neutraliser la formulation aqueuse à un pH supérieur ou égal à environ 6,5 et (d) à acidifier la formulation aqueuse à un pH se trouvant dans la plage allant d'environ 3 à 6,5.
